# EUROPEAN PATENT APPLICATION

(11) **EP 2 241 565 A1**
(43) Date of publication of application: **20.10.2010**
(21) Application number: 09382002.5
(22) Date of filing: 15.01.2009
(51) Int. Cl.: C07D 405/04, A61K 31/517, A61P 35/00

(54) **Aurora kinase inhibitors compounds**

(71) Applicant: Universität Leipzig, 04109 Leipzig (DE); Fundació Privada Centre de Regulació Genòmica (CRG), 08003 Barcelona (ES); Institució Catalana de Recerca i Estudis Avançats, 08010 Barcelona (ES)
(72) Inventor: Giannis, Athanassios, 04316 Leipzig (DE); Cottin, Thomas, 04249 Leipzig (DE); Sardón Urtiaga, Teresa, 08328 Alella (Barcelona) (ES); Vernos Ruscassier, Isabelle J., 08950 Esplugues de Llobregat (Barcelona) (ES)
(74) Representative: ZBM Patents

(57) **Abstract**

The present invention relates to novel compounds of formula (I) that inhibit cell proliferation, in particular by inhibiting selectively Aurora A kinase activity and to their use in medicine, in particular for treating, ameliorating or preventing hyperproliferative diseases. The invention further relate to methods of their preparation, pharmaceutical compositions including such compounds.

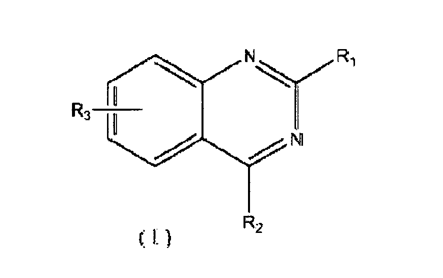

## Description

The present invention relates to the fields of chemistry and molecular pathology. More particularly, the present invention refers to novel compounds that inhibit cell proliferation, in particular by inhibiting Aurora A kinase activity and uses of these compounds for treating, ameliorating or preventing diseases, conditions or disorders benefiting from the inhibition of aurora activity, in particular hyperproliferative diseases.

### BACKGROUND ART

The formation of many tumors is related to overexpression of different phosphate-transferring enzymes, the kinases. For this reason the design of selective inhibitors became more important. One scaffold for this is the quinazoline, a flat aromatic molecule with two sp2-hybridized nitrogen-atoms in the ring being able to act as hydrogen bond acceptors and making the quinazoline an interesting lead structure for the search of ATP-analogue compounds. These compounds bind to the ATP-binding pocket of the kinases and avoid the binding of the phosphate-donor ATP. In this way the phosphorylation of different substrates can be avoided.

The cell cycle is essential for the proliferation of complex cell systems and includes four phases: G1 is the phase of cell growth, the S phase includes the identical replication of the DNA, the G2 phase prepares the cell for the fourth phase, the M phase or mitosis. In each phase different checkpoints control the system for possible damages and ensure that a phase doesn't begin before the previous has finished. The mitosis is also divided into different phases which are related to microscopically visible states of the cell. The prophase includes the movement of the centrosomes to the opposite cell poles and the chromosome condensation. In the prometaphase the nuclear envelope breakdown occurs. Microtubules capture the chromosomes and, throught the activity of motor proteins, move them towards the spindle equator. In the metaphase the sister chromatides show to opposite cell poles. The microtubules are attached to the kinetochores of the chromatides. The following anaphase is the phase of chromatide separation to the spindle poles. In the telophases the decondensation of the chromosomes occurs and a new nuclear envelope is formed. A contractile ring of actin filaments is formed and leads to the separation of the two daughter cells, the cytokinesis.

The formation of many tumors is related to uncontrolled cell division and overexpression of enzymes which are involved in cell cycle progression and the failure of different checkpoints. One related enzyme class is the class of the Aurora kinases, which play several functions at a number of events during G2- and M-phase. Due to the fact that their expression is higher at these cell stages, their inhibition should influence mainly dividing cells. The Aurora kinases are a family of serine/threonine kinases containing three members, Aurora A, B and C. They are highly conserved in the C-terminal region, where the kinase domain is located, and show sequence differences in the N-terminal domain (Nat. Rev. Cancer, 2005, 5, 42-49). Aurora B is expressed between the late G2-phase and telophase. It is located in the inner centromere region and in the spindle middle zone. It regulates the orientation of the chromosomes at the metaphase plate and corrects wrong kinetochore-microtubule interactions. It phosphorylates histone H3, which allows the histone to interact with the DNA. This is important for the following chromosome condensation. Aurora C shows high sequence homologies with Aurora B and has functions in the meiosis.

The expression of Aurora A kinase increases during G2-phase, peaking in the late G2-phaseand at the beginning of M-phase. The enzyme is located at the centrosomes, and after nuclear envelope breakdown it can also be observed on the mitotic spindle apparatus. For activation of Aurora A, phosphorylation of three different positions is necessary: Ser51, Thr288 and Ser342. Additionally the binding of the protein TPX2 increases its catalytic activity and at the same time it protects the enzyme against dephosphorylation of Thr288 and the related deactivation. It also decreases the accessibility of the enzyme for some inhibitors. During mitosis Aurora A is involved in different cellular processes i.a. the movement of the centrosomes to the opposite cell poles and the stabilization of the centrosomal microtubules by phosphorylation of a related protein complex. Aurora A is also involved in the entrance of the cell to mitosis. By phosphorylation Aurora A activates the phosphatase Cdc25 which dephosphorylates the kinases Cdk1 afterwards. This dephosphorylation is essential for the activation of the complex Cdk1/Cyclin B which is pivotal for the entrance of the cell to mitosis.

It is known that Aurora A is overexpressed in different tumor types e.g. of the bladder, breast, cervical, esophageal, gastric, kidney, laryngeal, prostate, pancreatic and leukemia. Aurora A overexpression occurs in early states of tumor development. Aurora A-overexpressing HeLa cells show chromosomes without correct order and free kinetochores in the metaphase. The start of the anaphase occurs despite the checkpoint of spindle formation is activated and leads to tetraploidy without cytokinesis. In cells showing also p53-deficiency the cell cycle goes on leading to aneuploidy which is one of the main origins of tumor formation. It was also shown that Aurora A phosphorylates the tumor suppressor protein p53. This phosphorylation increases the degradation of p53, so Aurora A overexpression can lead to increased p53-degradation. It was also shown that Aurora A-overexpression can lead to Taxol-resistance in HeLa cells.

At the moment some inhibitors of Aurora A exist, but no inhibitor is validated completely. Most of them do not show selectivity for Aurora A, but they also inhibit other kinases e.g. the related Aurora B.

To the known Aurora A inhibitors belongs the compound MLN8054, which is in phase I clinical trials but in one study it showed also Aurora B specific inhibition profiles when higher concentrations were administered. Another Aurora A inhibitor is MK-0457 (VX-680). It inhibits also Aurora B and Flt-3, and is therefore not Aurora A specific. Another Aurora A inhibitor, SU6668, shows low selectivity and inhibits other enzyme classes beside the Aurora kinases. After the results of the phase I studies it won't enter phase II studies. A known inhibitor based on the quinazoline scaffold is ZM447439 (hereinafter ZM1). It inhibits Aurora A in vitro with an IC₅₀ of 1000 nM, Aurora B with an IC₅₀ of 50 nM, so it shows no selectivity for Aurora A (Girdler et al., J. Cell Sci. 2006, 119, 3664-3675). So far no Aurora A inhibitors are launched, and no inhibitor shows strong selectivity for an inhibition of Aurora A in vitro as well as in vivo.

A number of quinazoline derivatives have been proposed hitherto for use in the inhibition of various kinases. For example, WO 01/21597, WO 96/09294, WO 96/15118 and WO 99/06378 describe the use of certain quinazoline compounds as receptor tyrosine kinase inhibitors, which may be useful in the treatment of proliferative disease and WO 00/21955 discloses certain quinazoline derivatives as inhibitors of the effects of VEGF.

EP 1147093 relates to 4-aminoquinazoline derivatives wherein no substitution in 2-position of the quinazoline moiety is present. The compounds described in EP 1147093 show certain activity as inhibitors of erbB family of protein kinases, such as EGFR, erbB2, HER3 or HER4.

EP 0579496 relates to 4-aminoquinazoline derivatives as inhibitors of cyclic guanosine-3',5'-monophosphate phosphodiesterase and simultaneously of thromboxane A2 synthetase. The compounds therein described are claimed to be particularly useful for the prevention and/or treatment of diseases induced by platelet aggregation, e.g. angina pectoris, heart failure, pulmonary hypertension and renal diseases. No reference is made to the use of the compounds therein described in the treatment of any kind of cancer. In EP 0579496 is cited the following compound: 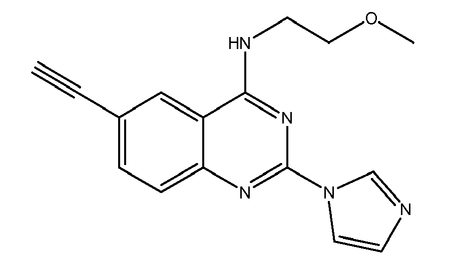 (6-ethynyl-2-(1H-imidazol-1-yl)-N-(2-methoxyethyl)quinazolin-4-amine). In a family related patent application, JP 07188214, is described the following compound: 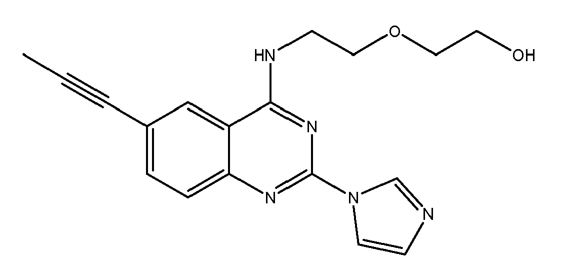 (2-(2-(2-(1H-imidazol-1-yl)-6-(prop-1-ynyl)quinazolin-4-ylamino)-ethoxy)ethanol).

WO 04/030671 relates to 4-aminoquinazoline derivatives as protein kinase B inhibitor, wherein the quinazoline moiety is substituted by a -(CH=CH)ₙ-R₄ radical in their 2-position. It is mentioned that the compounds therein described show an effect to one or more protein kinase B, preferably PKBα and/or PKBβ and/or PKBβ1 and/or PKBγ and/or PKBγ1.

However despite the compounds of therein described, there still exists the need for further compounds having Aurora kinase inhibitory properties as chemotherapeutic agents. More particularly compounds having Aurora A kinase inhibitory properties showing high selectivity for such kinase versus other Aurora kinases (B and C) and/or other protein kinases in general.

### SUMMARY OF THE INVENTION

The applicants have been successful in finding a novel series of compounds which inhibit the effects of the Aurora A kinase and which have certain properties that make them particularly useful in formulating medicaments for the treatment of disease. In particular, the compounds are useful in the treatment of proliferative diseases such as cancer. The compounds are specially useful in treating either solid or haematological tumours where Aurora kinases are known to be active, and especially in diseases such as colorectal, breast, lung, prostate, pancreatic or bladder and renal cancer as well as leukemias and lymphomas.

At the moment some Aurora A inhibitors exist, partly they show only low selectivity for the enzyme. So far no inhibitor is validated completely. The observed phenotype in cells treated with most of the known Aurora inhibitors, (although they show a promising specificity in *in vitro* assays) demonstrates that they inhibit preferably Aurora B over Aurora A *in vivo.*

It has been demonstrated that overexpression of Aurora A adversely affect to the activated spindle checkpoint for the paclitaxel or nocodazol treatment, inducing resistance to those drugs. Accordingly, the compounds of formula (I) allow to reduce or block the oncogenic activity of the protein and, additionally, improve the survival of oncologic patients with resistance to the above mentioned drugs.

Additionally, the compounds of formula (I) that specifically inhibit Aurora A and not B or C or other unrelated (at least not significantly) may be useful as a research tool, since these would allow a better understanding of the pathways and processes in that this protein is involved, as well as the consequences of its inactivation. Additionally, the existence of a Aurora A kinase inhibitor which shows a significant selectivity against other Aurora kinases and/or other protein kinases can be useful in screening of new chemotherapeutic agents.

The inventors of the present invention have found that the compounds of formula (I) of the present invention show a remarkable inhibitor activity against Aurora A kinase. Particularly, the compounds of the invention can optimally interact with the target protein. Owing to their optimal design the compounds of the invention can improve the affinity against the target protein, being possible avoiding the undesired side-effects related to other known Aurora kinase inhibitors. For instance, the dose needed to achieve the therapeutic effect compared with the doses used for other compounds of the state of the art can be reduced either reducing the frequency of administration or the dosage amount, making more comfortable to the patient the therapeutic regimen.

Thus, one aspect of the present invention relates to a compound of general formula (I), 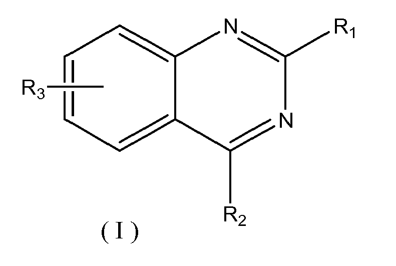 wherein:
R₁ is a radical of one of the known ring systems with 1-2 rings, wherein each one of the rings forming said ring system has 3-7 members, each member independently selected from C, N, O, S, CH, CH₂, NH,
   is saturated, partially unsaturated or aromatic, and
   is partially or totally fused; being each ring forming part of the ring system optionally substituted by at least one radical selected from the group consisting of: halogen, nitro, cyano, linear or branched (C₁-C₆)alkyl, halo-(C₁-C₆)alkyl, linear or branched (C₂-C₆)alkenyl, -OR₆, -COR₆, -COOR₆, -OC(O)R₆, -C(O)NR₄R₅, -NR₄R₅, -R₇NHR₈, -SR₆, -SO-R₆, and -SO₂-R₆;
   R₄, R₅, R₆, and R₈ are radicals, same or different, independently selected from the group consisting of hydrogen, and linear or branched (C₁-C₆)alkyl;
   R₇ is a biradical selected from the group consisting of: linear or branched (C₁-C₆)alkyl; linear or branched (C₂-C₆)alkenyl; and linear or branched (C₂-C₆)alkynyl;
   R₂ is a radical selected from the group consisting of -NHR₉, -C≡C-CR₁₀R₁₁R₁₂, (*E*)-CH=CH-CR₁₀R₁₁R₁₂, and (Z)-CH=CH-CR₁₀R₁₁R₁₂;
   R₉ is a radical selected from the group consisting of:

linear or branched (C₁-C₆)alkyl optionally substituted by at least one radical selected from the group consisting of halogen, -OH, -SH, nitro, cyano, -NH₂, linear or branched (C₁-C₄)alkyl, halo-(C₁-C₆)alkyl, -O-(C₁-C₆)alkyl, -S-(C₁-C₆)alkyl, -COR₁₃, -COOR₁₃, -OC(O)R₁₃, -SOR₁₃, -SOOR₁₃, -OS(O)R₁₃; and
a known ring system having 3-6 members, each member independently selected from C, N, O, S, CH, CH₂, and NH, wherein the ring is saturated, partially unsaturated or aromatic, optionally substituted by at least one radical selected from the group consisting of halogen, -OH, -SH, nitro, cyano, amino, linear or branched (C₁-C₄)alkyl, halo-(C₁-C₆)alkyl, -O-(C₁-C₆)alkyl, -S-(C₁-C₆)alkyl, -COR₁₄, -COOR₁₄, -OC(O)R₁₄, -SOR₁₄, -SOOR₁₄, and -OS(O)R₁₄;
R₁₀, R₁₁, and R₁₂ are radicals, same or different, independently selected from the group consisting of hydrogen, halogen, linear or branched (C₁-C₆)alkyl, -NH₂, -OH, -SH, -NR₁₅R₁₆, halo-(C₁-C₆)alkyl, -OR₁₇, -SR₁₇, -COR₁₇, -COOR₁₇, -OC(O)R₁₇, -SOR₁₇, -SOOR₁₇, -OS(O)R₁₇, and -C(O)NR₁₅R₁₆;
R₁₃, R₁₄, R₁₅, R₁₆ and R₁₇ are radicals, same or different, independently selected from the group consisting of hydrogen, and linear or branched (C₁-C₄)alkyl;
R₃ is a radical selected from the group consisting of -C≡C-CR₁₈R₁₉R₂₀, (*E*)-CH=CH-CR₁₈R₁₉R₂₀, and (*Z*)-CH=CH-CR₁₈R₁₉R₂₀;
R₁₈, R₁₉, and R₂₀ are radicals, same or different, independently selected from the group consisting of hydrogen, halogen, linear or branched (C₁-C₆)alkyl, -NH₂, -OH, -SH, -NR₂₁R₂₂, halo-(C₁-C₆)alkyl, -OR₂₃, -SR₂₃, -COR₂₃, -COOR₂₃, -OC(O)R₂₃, -SOR₂₃, -SOOR₂₃, -OS(O)R₂₃, and -C(O)NR₂₁R₂₂;
R₂₁, R₂₂, and R₂₃ are radicals, same or different, independently selected from the group consisting of hydrogen, and linear or branched (C₁-C₄)alkyl;
   with the proviso that it is not
   6-ethynyl-2-(1H-imidazol-1-yl)-N-(2-methoxyethyl)quinazolin-4-amine or 2-(2-(2-(1H-imidazol-1-yl)-6-(prop-1-ynyl)quinazolin-4-ylamino)ethoxy)ethanol

The kinase domain of the three Aurora kinases shows high sequence similarities and a lot of similarities with this of other kinases. It is believed that the compounds of the invention are able to bind selectively to the so-called fluorophenyl pocket of the ATP binding site of the catalytic domain of Aurora A to inhibit the kinase function, owing to the proper combination of the different radicals R₁-R₃ on the quinazoline moiety, and their three-dimensional disposition. Regarding the chemical nature of the susbtituents R₁, R₂ and R₃, it has been found that the selectivity of the compounds is affected especially by the R₁ position which may affect the binding affinity, and hence the activity.

A second aspect of the invention is a pharmaceutical composition comprising a therapeutically effective amount of a compound of formula (I) according to the first aspect of the invention, together with the appropriate amounts of pharmaceutically acceptable excipients, carriers, or mixtures thereof.

A third aspect of the invention provides a compound according to the first aspect of the invention for use as a medicament.

A fourth aspect of the invention is the use of a compound of formula (I), including
6-ethynyl-2-(1H-imidazol-1-yl)-N-(2-methoxyethyl)quinazolin-4-amine or 2-(2-(2-(1H-imidazol-1-yl)-6-(prop-1-ynyl)quinazolin-4-ylamino)ethoxy)ethanol , for the manufacture of a medicament for the treatment of a disease mediated by Aurora A kinase. This aspect of the invention can also be formulated as compound of formula (I) as defined in the fourth aspect of the invention for use in the treatment of a disease mediated by a Aurora A kinases.

### DETAILED DESCRIPTION OF THE INVENTION:

According to the present invention when the ring system is formed by "isolated" rings means that the ring system is formed by two, three or four rings and said rings are bound via a bond from the atom of one ring to the atom of the other ring.

The term "isolated" also embraces the embodiment in which the ring system has only one ring. Illustrative non-limitative examples of known ring systems consisting of one ring are those selected from: cyclopropyl, cyclobutyl, cyclopentyl, cyclohexyl, cycloheptyl, cyclopropenyl, cyclobutenyl, cyclopentenyl, phenyl, cycloheptenyl, aziridinyl, oxirenyl, thiiranyl, azetidinyl, oxetanyl, pyrrolyl, furanyl, thiophenyl, pyrazolyl, imidazolyl, triazolyl, tetrazolyl, oxazolyl, isoxazolyl, thiazolyl, isothiazolyl, dithiolanyl, pyridinyl, pyranyl, pyridazinyl, pyrimidinyl, pyrazinyl, piperazinyl, oxazinyl, thiazinyl, dithianyl, and dioxanyl.

According to the present invention when the ring system has rings "totally fused", means that the ring system is formed by two rings in which two or more atoms are common to two adjoining rings. Illustrative non-limitative examples of known ring systems consisting of two rings totally fused, are those selected from benzofuran, isobenzofuran, indole, isoindole, indolizine, indoline, isoindoline, purine (e.g., adenine, guanine), benzimidazole, indazole, benzoxazole, benzisoxazole, benzodioxole, benzofurazan, benzotriazole, benzothiofuran, benzothiazole, benzothiadiazole, heterocyclic chromene, isochromene, chroman, isochroman, benzodioxan, quinoline, isoquinoline, quinolizine, benzoxazine, benzodiazine, pyridopyridine, quinoxaline, quinazoline, cinnoline, phthalazine, naphthyridine, pteridine.

According to the present invention when the ring system is "partially fused" it means that the ring system is formed by three or four rings, being at least two of said rings totally fused (i.e. two or more atoms being common to the two adjoining rings) and the remaining ring(s) being bound via a bond from the atom of one ring to the atom of one of the fused rings.

According to an embodiment of the first aspect of the invention, the compound of formula (I) is one wherein:
R₁ is a radical of a known ring system consisting of one ring which is saturated, partially unsaturated or aromatic, has from 5-6 members, each member is independently selected from C, N, O, S, CH, CH₂, NH; being the radical optionally substituted by at least one radical selected from the group consisting of: halogen, nitro, cyano, linear or branched (C₁-C₆)alkyl, halo-(C₁-C₆)alkyl, linear or branched (C₂-C₆)alkenyl, -OR₆, -COR₆, -COOR₆, -OC(O)R₆, -C(O)NR₄R₅, -NR₄R₅, -R₇NHR₈, -SR₆, -SO-R₆, and -SO₂-R₆;
R₄, R₅, R₆, and R₈ are radicals, same or different, independently selected from the group consisting of hydrogen, and linear or branched (C₁-C₆)alkyl;
R₇ is a biradical selected from the group consisting of: linear or branched (C₁-C₆)alkyl; linear or branched (C₂-C₆)alkenyl; and linear or branched (C₂-C₆)alkynyl.

According to another embodiment of the first aspect of the invention, the compound of formula (I) is one wherein:
R₁ is a radical selected from 2-furyl, 3-furyl, 2-thiophenyl, 3-thiophenyl, 2-thiazolyl, 4-thiazolyl, 5-thiazolyl, 2-oxazolyl, 4-oxazolyl, 5-oxazolyl, 3-isothiazolyl, 4-isothiazolyl, 5-isothiazolyl, 2-pyrrolyl, 3-pyrrolyl, 2-imidazolyl, 4-imidazolyl, 5-imidazolyl, 1-pyrazolyl, 3-isoxazolyl, 4-isoxazolyl, 2-pyridinyl, 3-pyridinyl, 4-pyridinyl, pyrazinyl, and phenyl; being the radical optionally substituted by at least one radical selected from the group consisting of: halogen, nitro, cyano, linear or branched (C₁-C₆)alkyl, halo-(C₁-C₆)alkyl, linear or branched (C₂-C₆)alkenyl, -OR₆, -COR₆, -COOR₆, -OC(O)R₆, -C(O)NR₄R₅, -NR₄R₅, -R₇NHR₈, -SR₆, -SO-R₆, and -SO₂-R₆;
R₄, R₅, R₆, and R₈ are radicals, same or different, independently selected from the group consisting of hydrogen, and linear or branched (C₁-C₆)alkyl;
R₇ is a biradical selected from the group consisting of: linear or branched (C₁-C₆)alkyl; linear or branched (C₂-C₆)alkenyl; and linear or branched (C₂-C₆)alkynyl.

According to another embodiment of the first aspect of the invention, the compound of formula (I) is one wherein:
R₁ is a radical selected from 2-furyl, 3-furyl, 2-thiophenyl, 3-thiophenyl, 2-thiazolyl, 4-thiazolyl, 5-thiazolyl, 2-oxazolyl, 4-oxazolyl, 5-oxazolyl, 3-isothiazolyl, 4-isothiazolyl, 5-isothiazolyl, 2-pyrrolyl, 3-pyrrolyl, 3-isoxazolyl, 4-isoxazolyl, and phenyl; being the radical optionally substituted by at least one radical selected from the group consisting of: halogen, nitro, cyano, linear or branched (C₁-C₆)alkyl, halo-(C₁-C₆)alkyl, linear or branched (C₂-C₆)alkenyl, -OR₆, -COR₆, -COOR₆, -OC(O)R₆, -C(O)NR₄R₅, -NR₄R₅, -R₇NHR₈, -SR₆, -SO-R₆, and -SO₂-R₆;
R₄, R₅, R₆, and R₈ are radicals, same or different, independently selected from the group consisting of hydrogen, and linear or branched (C₁-C₆)alkyl;
R₇ is a biradical selected from the group consisting of: linear or branched (C₁-C₆)alkyl; linear or branched (C₂-C₆)alkenyl; and linear or branched (C₂-C₆)alkynyl.

In a more preferred embodiment of the invention, R₁ is a radical selected from 2-furyl, 3-furyl, 2-thiophenyl, and 3-thiophenyl; being the radical optionally substituted by at least one radical selected from the group consisting of: halogen, nitro, cyano, linear or branched (C₁-C₆)alkyl, halo-(C₁-C₆)alkyl, linear or branched (C₂-C₆)alkenyl, -OR₆, -COR₆, -COOR₆, -OC(O)R₆, -C(O)NR₄R₅, -NR₄R₅, -R₇NHR₈, -SR₆, -SO-R₆, and -SO₂-R₆;
R₄, R₅, R₆, and R₈ are radicals, same or different, independently selected from the group consisting of hydrogen, and linear or branched (C₁-C₆)alkyl;
R₇ is a biradical selected from the group consisting of: linear or branched (C₁-C₆)alkyl; linear or branched (C₂-C₆)alkenyl; and linear or branched (C₂-C₆)alkynyl.

In still yet another embodiment of the first aspect of the invention, the compound of formula (I) is one wherein the substituents of R₁, if present, are selected from the group consisting of: fluorine, chlorine, bromine, iodine, methyl, ethyl, propyl, isopropyl, trifluoromethyl, and -OH.

In another embodiment of the first aspect of the invention, the compound of formula (I) is one wherein:
R₂ is a -NHR₉; being R₉ a radical selected from the group consisting of:
   linear or branched (C₁-C₆)alkyl optionally substituted by at least one radical selected from the group consisting of halogen, -OH, -SH, nitro, cyano, -NH₂, (C₁-C₄)alkyl, halo-(C₁-C₆)alkyl, -O-(C₁-C₆)alkyl, -S-(C₁-C₆)alkyl, -COR₁₃, -COOR₁₃, -OC(O)R₁₃, -SOR₁₃, -SOOR₁₃, and -OS(O)R₁₃; and
   a known ring system having 3-6 members, each member independently selected from C, N, O, S, CH, CH₂, and NH, wherein the ring is saturated, partially unsaturated or aromatic, optionally substituted by at least one radical selected from the group consisting of halogen, -OH, -SH, nitro, cyano, amino, linear or branched (C₁-C₄)alkyl, halo-(C₁-C₆)alkyl, -O-(C₁-C₆)alkyl, -S-(C₁-C₆)alkyl, -COR₁₄, -COOR₁₄, -OC(O)R₁₄, -SOR₁₄, -SOOR₁₄, and -OS(O)R₁₄;
R₁₃, and R₁₄ are radicals, same or different, independently selected from the group consisting of hydrogen, and linear or branched (C₁-C₄)alkyl.

In still another embodiment, the compound is one wherein:
R₂ is a -NHR₉; being R₉ a radical selected from the group consisting of:
   methyl, ethyl, propyl, isopropyl, butyl, isobutyl, s-butyl, t-butyl, cyclopropyl, cyclobutyl, cyclopentyl, cyclohexyl, 2H-pyran-3-yl, 2-thienyl, 3-thienyl, 2-furyl, 3-furyl, 2-thiophenyl, 3-thiophenyl, 2-thiazole, 4-thiazole, 5-thiazole, 2-oxazole, 4-oxazole, 5-oxazole, 3-isothiazole, 4-isothiazole, 5-isothiazole, 2-pyrrole, 3-pyrrole, 2-imidazole, 4-imidazole, 5-imidazole, 1-pyrazolyl, 3-pyrazolyl, 2-pyridyl, 3-pyridyl, 4-pyridyl, pyrazinyl, 2-pyrimidinyl, and phenyl; being the radical optionally substituted by at least one radical selected from the group consisting of halogen, -OH, -SH, nitro, cyano, amino, linear or branched (C₁-C₄)alkyl, halo-(C₁-C₆)alkyl, -O-(C₁-C₆)alkyl, -S-(C₁-C₆)alkyl, -COH, -COOH, -CO(C₁-C₄)alkyl, -COO(C₁-C₄)alkyl, -OC(O)(C₁-C₄)alkyl, -SOH, -SO₂H, -SO(C₁-C₄)alkyl, -SO₂(C₁-C₄)alkyl, -OS(O)(C₁-C₄)alkyl.

In still yet another embodiment of the invention, R₂ is a -NHR₉; being R₉ a radical selected from the group consisting of:
methyl, ethyl, propyl, isopropyl, butyl, isobutyl, s-butyl, t-butyl, cyclopropyl, cyclobutyl, cyclopentyl, cyclohexyl, and phenyl; being the radical optionally substituted by at least one radical selected from the group consisting of halogen, -OH, -SH, nitro, cyano, amino, linear or branched (C₁-C₄)alkyl, halo-(C₁-C₆)alkyl, -O-(C₁-C₆)alkyl,-S-(C₁-C₆)alkyl, -COH, -COOH, -CO(C₁-C₄)alkyl, -COO(C₁-C₄)alkyl, -OC(O)(C₁-C₄)alkyl, -SOH, -SO₂H, -SO(C₁-C₄)alkyl, -SO₂(C₁-C₄)alkyl, -OS(O)(C₁-C₄)alkyl;

According to another embodiment of the invention, R₂ is a radical selected from the group consisting -C≡C-CR₁₀R₁₁R₁₂, (*E*)-CH=CH-CR₁₀R₁₁R₁₂, and (*Z*)-CH=CH-CR₁₀R₁₁R₁₂; wherein:
R₁₀, R₁₁, and R₁₂ are radicals, same or different, independently selected from the group consisting of hydrogen, halogen, linear or branched (C₁-C₆)alkyl, -NH₂, -OH, -SH, -NR₁₅R₁₆, halo-(C₁-C₆)alkyl, -OR₁₇, -SR₁₇, -COR₁₇, -COOR₁₇, -OC(O)R₁₇, -SOR₁₇, -SOOR₁₇, -OS(O)R₁₇, and -C(O)NR₁₅R_{16;}

R₁₅, R₁₆ and R₁₇ are radicals, same or different, independently selected from the group consisting of hydrogen, and linear or branched (C₁-C₄)alkyl.

In yet another embodiment, R₂ is a radical selected from the group consisting of -C≡C-CR₁₀R₁₁R₁₂, (*E*)-CH=CH-CR₁₀R₁₁R₁₂, and (*Z*)-CH=CH-CR₁₀R₁₁R₁₂; wherein:
at least one of R₁₀, R₁₁ and R₁₂ is hydrogen; and the other R₁₀, R₁₁ or R₁₂ are radicals, same or different, independently selected from the group consisting of hydrogen, halogen, methyl, ethyl, propyl, isopropyl, butyl, isobutyl, s-butyl, t-butyl, -OH, -SH, -NR₁₃R₁₄, trifluoromethyl, trifluoroethyl, methoxyl, ethoxyl, thiomethyl, thioethyl, -COH, -COOH, -COCH₃, -COOCH₃, -OC(O)CH₃, -SOH, -SO₂H, -SOCH₃, -SO₂CH₃, -OS(O)CH₃, and -C(O)NR₁₃R_{14;}
being R₁₃ and R₁₄, same or different, independently selected from hydrogen, methyl, ethyl, propyl, and isopropyl.

In still yet another embodiment, R₂ is a radical selected from the group consisting of -C≡C-CH₂CH₃, -C≡C-CH(CH₃)₂, -C≡C-CH₂CH₂OH, -C≡C-CH₂CH₂OH, -C≡C-CH₂COOH, -C≡C-CH₂CHO, -C≡C-CH₂CH₂CH₃, -C≡C-CH₂CH(CH₃)₂, -C≡C-CF₃, -C≡C-CH₂SH, -C≡C-CH₂NH₂, -C≡C-CH₂N(CH₃)₂, -C≡C-CH₂SOCH₃, -C≡C-CH₂SO₂CH₃, -C≡C-CH₂COOCH₃, (*E*)-CH=CH-CH₂CH₃, (*E*)-CH=CH-CH₂OH, (*E*)-CH=CH-CH₂SH, (*E*)-CH=CH-CH₂NH₂, (*E*)-CH=CH-CH(CH₃)₂, (*E*)-CH=CH-CHCH₃OH, (*E*)-CH=CH-CHCH₃SH, (*E*)-CH=CH-CHCH₃NH₂, (*E*)-CH=CH-C(CH₃)₃, (*E*)-CH=CH-C(CH₃)₂OH, (*E*)-CH=CH-C(CH₃)₂SH, (*E*)-CH=CH-C(CH₃)₂NH₂, (*E*)-CH=CH-CH₂COOH, (*E*)-CH=CH-CH₂COOC(CH₃)₃, (*E*)-CH=CH-CH₂N(CH₃)₂, (*E*)-CH=CH-CH₂COOCH₃, (*Z*)-CH=CH-CH₂CH₃, (*Z*)-CH=CH-CH₂OH, (*Z*)-CH=CH-CH₂SH, (*Z*)-CH=CH-CH₂NH₂, (*Z*)-CH=CH-CH(CH₃)₂, (*Z*)-CH=CH-CHCH₃0H, (*Z*)-CH=CH-CHCH₃SH, (*Z*)-CH=CH-CHCH₃NH₂, (*Z*)-CH=CH-C(CH₃)₃, (*Z*)-CH=CH-C(CH₃)₂OH, (*Z*)-CH=CH-C(CH₃)₂SH, (*Z*)-CH=CH-C(CH₃)₂NH₂, (*Z*)-CH=CH-CH₂COOH, (*Z*)-CH=CH-CH₂COOC(CH₃)₃, (*Z*)-CH=CH-CH₂N(CH₃)₂, and (*Z*)-CH=CH-CH₂COOCH₃.

According to another embodiment, R₃ is a radical selected from the group consisting of -C≡C-CR₁₈R₁₉R₂₀, (*E*)-CH=CH-CR₁₈R₁₉R₂₀, and (*Z*)-CH=CH-CR₁₈R₁₉R₂₀, wherein:
at least one of R₁₈, R₁₉ and R₂₀ is hydrogen; and the other R₁₈, R₁₉ or R₂₀ are radicals, same or different, independently selected from the group consisting of hydrogen, halogen, methyl, ethyl, propyl, isopropyl, butyl, isobutyl, s-butyl, t-butyl, -OH, -SH, -NR₂₁R₂₂, trifluoromethyl, trifluoroethyl, methoxyl, ethoxyl, thiomethyl, thioethyl, -COH, -COOH, -COCH₃, -COOCH₃, -OC(O)CH₃, -SOH, -SO₂H, -SOCH₃, -SO₂CH₃, -OS(O)CH₃, and -C(O)NR₂₁R₂₂;
R₂₁ and R₂₂ are radicals, same or different, independently selected from the group consisting of hydrogen, and linear or branched (C₁-C₄)alkyl.

In still yet another embodiment, R₃ is a radical selected from the group consisting of -C≡C-CH₂CH₃, -C≡C-CH(CH₃)₂, -C≡C-CH₂CH₂OH, -C≡C-CH₂CH₂OH, -C≡C-CH₂COOH, -C≡C-CH₂CHO, -C≡C-CH₂CH₂CH₃, -C≡C-CH₂CH(CH₃)₂, -C≡C-CF₃, -C≡C-CH₂SH, -C≡C-CH₂NH₂, -C≡C-CH₂N(CH₃)₂, -C≡C-CH₂SOCH₃, -C≡C-CH₂SO₂CH₃, -C≡C-CH₂COOCH₃, (*E*)-CH=CH-CH₂CH₃, (*E*)-CH=CH-CH₂OH, (*E*)-CH=CH-CH₂SH, (*E*)-CH=CH-CH₂NH₂, (*E*)-CH=CH-CH(CH₃)₂, (*E*)-CH=CH-CHCH₃0H, (*E*)-CH=CH-CHCH₃SH, (*E*)-CH=CH-CHCH₃NH₂, (*E*)-CH=CH-C(CH₃)₃, (*E*)-CH=CH-C(CH₃)₂OH, (*E*)-CH=CH-C(CH₃)₂SH, (*E*)-CH=CH-C(CH₃)₂NH₂, (*E*)-CH=CH-CH₂COOH, (*E*)-CH=CH-CH₂COOC(CH₃)₃, (*E*)-CH=CH-CH₂N(CH₃)₂, (*E*)-CH=CH-CH₂COOCH₃, (*Z*)-CH=CH-CH₂CH₃, (*Z*)-CH=CH-CH₂OH, (*Z*)-CH=CH-CH₂SH, (*Z*)-CH=CH-CH₂NH₂, (*Z*)-CH=CH-CH(CH₃)₂, (*Z*)-CH=CH-CHCH₃0H, (*Z*)-CH=CH-CHCH₃SH, (*Z*)-CH=CH-CHCH₃NH₂, (*Z*)-CH=CH-C(CH₃)₃, (*Z*)-CH=CH-C(CH₃)₂OH, (*Z*)-CH=CH-C(CH₃)₂SH, (*Z*)-CH=CH-C(CH₃)₂NH₂, (*Z*)-CH=CH-CH₂COOH, (*Z*)-CH=CH-CH₂COOC(CH₃)₃, (*Z*)-CH=CH-CH₂N(CH₃)₂, and (*Z*)-CH=CH-CH₂COOCH₃.

According to another embodiment of the first aspect of the invention, the compound of formula (I) is one wherein:
R₁ is a radical selected from 2-furyl, 3-furyl, 2-thiophenyl, 3-thiophenyl, 2-thiazolyl, 4-thiazolyl, 5-thiazolyl, 2-oxazolyl, 4-oxazolyl, 5-oxazolyl, 3-isothiazolyl, 4-isothiazolyl, 5-isothiazolyl, 2-pyrrolyl, 3-pyrrolyl, 3-isoxazolyl, 4-isoxazolyl and phenyl; being the radical optionally substituted by at least one radical selected from the group consisting of: halogen, nitro, cyano, linear or branched (C₁-C₆)alkyl, halo-(C₁-C₆)alkyl, linear or branched (C₂-C₆)alkenyl, -OR₆, -COR₆, -COOR₆, -OC(O)R₆, -C(O)NR₄R₅, -NR₄R₅, -R₇NHR₈, -SR₆, -SO-R₆, and -SO₂-R₆;
R₄, R₅, R₆, and R₈ are radicals, same or different, independently selected from the group consisting of hydrogen, and linear or branched (C₁-C₆)alkyl;
R₇ is a biradical selected from the group consisting of: linear or branched (C₁-C₆)alkyl; linear or branched (C₂-C₆)alkenyl; and linear or branched (C₂-C₆)alkynyl;
R₂ is a -NHR₉; being R₉ a radical selected from the group consisting of:
   methyl, ethyl, propyl, isopropyl, butyl, isobutyl, s-butyl, t-butyl, cyclopropyl, cyclobutyl, cyclopentyl, cyclohexyl, 2H-pyran-3-yl, 2-thienyl, 3-thienyl, 2-furyl, 3-furyl, 2-thiophenyl, 3-thiophenyl, 2-thiazole, 4-thiazole, 5-thiazole, 2-oxazole, 4-oxazole, 5-oxazole, 3-isothiazole, 4-isothiazole, 5-isothiazole, 2-pyrrole, 3-pyrrole, 2-imidazole, 4-imidazole, 5-imidazole, 1-pyrazolyl, 3-pyrazolyl, 2-pyridyl, 3-pyridyl, 4-pyridyl, pyrazinyl, 2-pyrimidinyl, and phenyl; being the radical optionally substituted by at least one radical selected from the group consisting of halogen, -OH, -SH, nitro, cyano, amino, linear or branched (C₁-C₄)alkyl, halo-(C₁-C₆)alkyl, -O-(C₁-C₆)alkyl, -S-(C₁-C₆)alkyl, -COH, -COOH, -CO(C₁-C₄)alkyl, -COO(C₁-C₄)alkyl, -OC(O)(C₁-C₄)alkyl, -SOH, -SO₂H, -SO(C₁-C₄)alkyl, -SO₂(C₁-C₄)alkyl, -OS(O)(C₁-C₄)alkyl;
R₃ is a radical selected from the group consisting of -C≡C-CR₁₈R₁₉R₂₀, (*E*)-CH=CH-CR₁₈R₁₉R₂₀, and (*Z*)-CH=CH-CR₁₈R₁₉R₂₀, wherein:
   at least one of R₁₈, R₁₉ and R₂₀ is hydrogen; and the other R₁₈, R₁₉ or R₂₀ are radicals, same or different, independently selected from the group consisting of hydrogen, halogen, methyl, ethyl, propyl, isopropyl, butyl, isobutyl, s-butyl, t-butyl, -OH, -SH, -NR₂₁R₂₂, trifluoromethyl, trifluoroethyl, methoxyl, ethoxyl, thiomethyl, thioethyl, -COH, -COOH, -COCH₃, -COOCH₃, -OC(O)CH₃, -SOH, -SO₂H, -SOCH₃, -SO₂CH₃, -OS(O)CH₃, and -C(O)NR₂₁R₂₂;
R₂₁ and R₂₂ are radicals, same or different, independently selected from the group consisting of hydrogen, and linear or branched (C₁-C₄)alkyl.

According to another preferred embodiment of the first aspect of the invention, the compound of formula (I) is one wherein:
R₁ is a radical selected from 2-furyl, 3-furyl, 2-thiophenyl, and 3-thiophenyl; being the radical optionally substituted by at least one radical selected from the group consisting of: halogen, nitro, cyano, linear or branched (C₁-C₆)alkyl, halo-(C₁-C₆)alkyl, linear or branched (C₂-C₆)alkenyl, -OR₆, -COR₆, -COOR₆, -OC(O)R₆, -C(O)NR₄R₅, -NR₄R₅, -R₇NHR₈, -SR₆, -SO-R₆, and -SO₂-R₆;
R₄, R₅, R₆, and R₈ are radicals, same or different, independently selected from the group consisting of hydrogen, and linear or branched (C₁-C₆)alkyl;
R₇ is a biradical selected from the group consisting of: linear or branched (C₁-C₆)alkyl; linear or branched (C₂-C₆)alkenyl; and linear or branched (C₂-C₆)alkynyl;
R₂ is a -NHR₉; being R₉ a radical selected from the group consisting of:
   methyl, ethyl, propyl, isopropyl, butyl, isobutyl, s-butyl, t-butyl, cyclopropyl, cyclobutyl, cyclopentyl, cyclohexyl, and phenyl; being the radical optionally substituted by at least one radical selected from the group consisting of halogen, -OH, -SH, nitro, cyano, amino, linear or branched (C₁-C₄)alkyl, halo-(C₁-C₆)alkyl, -O-(C₁-C₆)alkyl, -S-(C₁-C₆)alkyl, -COH, -COOH, -CO(C₁-C₄)alkyl, -COO(C₁-C₄)alkyl, -OC(O)(C₁-C₄)alkyl, -SOH, -SO₂H, -SO(C₁-C₄)alkyl, -SO₂(C₁-C₄)alkyl, -OS(O)(C₁-C₄)alkyl;
R₃ is a radical selected from the group consisting of -C≡C-CR₁₈R₁₉R₂₀, (*E*)-CH=CH-CR₁₈R₁₉R₂₀, and (*Z*)-CH=CH-CR₁₈R₁₉R₂₀, wherein:
   at least one of R₁₈, R₁₉ and R₂₀ is hydrogen; and the other R₁₈, R₁₉ or R₂₀ are radicals, same or different, independently selected from the group consisting of hydrogen, halogen, methyl, ethyl, propyl, isopropyl, butyl, isobutyl, s-butyl, t-butyl, -OH, -SH, -NR₂₁R₂₂, trifluoromethyl, trifluoroethyl, methoxyl, ethoxyl, thiomethyl, thioethyl, -COH, -COOH, -COCH₃, -COOCH₃, -OC(O)CH₃, -SOH, -SO₂H, -SOCH₃, -SO₂CH₃, -OS(O)CH₃, and -C(O)NR₂₁R₂₂;
R₂₁ and R₂₂ are radicals, same or different, independently selected from the group consisting of hydrogen, and linear or branched (C₁-C₄)alkyl.

According to another embodiment of the first aspect of the invention, the compound of formula (I) is one wherein:
R₁ is a radical selected from 2-furyl, 3-furyl, 2-thiophenyl, 3-thiophenyl, 2-thiazolyl, 4-thiazolyl, 5-thiazolyl, 2-oxazolyl, 4-oxazolyl, 5-oxazolyl, 3-isothiazolyl, 4-isothiazolyl, 5-isothiazolyl, 2-pyrrolyl, 3-pyrrolyl, 3-isoxazolyl, 4-isoxazolyl and phenyl; being the radical optionally substituted by at least one radical selected from the group consisting of: halogen, nitro, cyano, linear or branched (C₁-C₆)alkyl, halo-(C₁-C₆)alkyl, linear or branched (C₂-C₆)alkenyl, -OR₆, -COR₆, -COOR₆, -OC(O)R₆, -C(O)NR₄R₅, -NR₄R₅, -R₇NHR₈, -SR₆, -SO-R₆, and -SO₂-R₆;
R₄, R₅, R₆, and R₈ are radicals, same or different, independently selected from the group consisting of hydrogen, and linear or branched (C₁-C₆)alkyl;
R₇ is a biradical selected from the group consisting of: linear or branched (C₁-C₆)alkyl; linear or branched (C₂-C₆)alkenyl; and linear or branched (C₂-C₆)alkynyl;
R₂ is a radical selected from the group consisting of -C≡C-CR₁₀R₁₁R₁₂, (*E*)-CH=CH-CR₁₀R₁₁R₁₂, and (*Z*)-CH=CH-CR₁₀R₁₁R₁₂; wherein:
R₁₀, R₁₁, and R₁₂ are radicals, same or different, independently selected from the group consisting of hydrogen, halogen, linear or branched (C₁-C₆)alkyl, -NH₂, -OH, -SH, -NR₁₅R₁₆, halo-(C₁-C₆)alkyl, -OR₁₇, -SR₁₇, -COR₁₇, -COOR₁₇, -OC(O)R₁₇, -SOR₁₇, -SOOR₁₇, -OS(O)R₁₇, and -C(O)NR₁₅R₁₆;
R₁₅, R₁₆ and R₁₇ are radicals, same or different, independently selected from the group consisting of hydrogen, and linear or branched (C₁-C₄)alkyl;
R₃ is a radical selected from the group consisting of -C≡C-CR₁₈R₁₉R₂₀, (*E*)-CH=CH-CR₁₈R₁₉R₂₀, and (*Z*)-CH=CH-CR₁₈R₁₉R₂₀, wherein:
   at least one of R₁₈, R₁₉ and R₂₀ is hydrogen; and the other R₁₈, R₁₉ or R₂₀ are radicals, same or different, independently selected from the group consisting of hydrogen, halogen, methyl, ethyl, propyl, isopropyl, butyl, isobutyl, s-butyl, t-butyl, -OH, -SH, -NR₂₁R₂₂, trifluoromethyl, trifluoroethyl, methoxyl, ethoxyl, thiomethyl, thioethyl, -COH, -COOH, -COCH₃, -COOCH₃, -OC(O)CH₃, -SOH, -SO₂H, -SOCH₃, -SO₂CH₃, -OS(O)CH₃, and -C(O)NR₂₁R₂₂;
R₂₁ and R₂₂ are radicals, same or different, independently selected from the group consisting of hydrogen, and linear or branched (_{C}1-C₄)alkyl.

According to another preferred embodiment of the first aspect of the invention, the compound of formula (I) is one wherein:
R₁ is a radical selected from 2-furyl, 3-furyl, 2-thiophenyl, and 3-thiophenyl; being the radical optionally substituted by at least one radical selected from the group consisting of: halogen, nitro, cyano, linear or branched (C₁-C₆)alkyl, halo-(C₁-C₆)alkyl, linear or branched (C₂-C₆)alkenyl, -OR₆, -COR₆, -COOR₆, -OC(O)R₆, -C(O)NR₄R₅, -NR₄R₅, -R₇NHR₈, -SR₆, -SO-R₆, and -SO₂-R₆;
R₄, R₅, R₆, and R₈ are radicals, same or different, independently selected from the group consisting of hydrogen, and linear or branched (C₁-C₆)alkyl;
R₇ is a biradical selected from the group consisting of: linear or branched (C₁-C₆)alkyl; linear or branched (C₂-C₆)alkenyl; and linear or branched (C₂-C₆)alkynyl;
R₂ is a radical selected from the group consisting of -C≡C-CR₁₀R₁₁R_{12;} wherein:
   at least one of R₁₀, R₁₁ and R₁₂ is hydrogen; and the other R₁₀, R₁₁ or R₁₂ are radicals, same or different, independently selected from the group consisting of hydrogen, halogen, methyl, ethyl, propyl, isopropyl, butyl, isobutyl, s-butyl, t-butyl, -OH, -SH, -NR₁₃R₁₄, trifluoromethyl, trifluoroethyl, methoxyl, ethoxyl, thiomethyl, thioethyl, -COH, -COOH, -COCH₃, -COOCH₃, -OC(O)CH₃, -SOH, -SO₂H, -SOCH₃, -SO₂CH₃, -OS(O)CH₃, and -C(O)NR₁₃R_{14;}
   being R₁₃ and R₁₄, same or different, independently selected from hydrogen, methyl, ethyl, propyl, and isopropyl;
R₃ is a radical selected from the group consisting of -C≡C-CR₁₈R₁₉R₂₀, (*E*)-CH=CH-CR₁₈R₁₉R₂₀, and (*Z*)-CH=CH-CR₁₈R₁₉R₂₀, wherein:
   at least one of R₁₈, R₁₉ and R₂₀ is hydrogen; and the other R₁₈, R₁₉ or R₂₀ are radicals, same or different, independently selected from the group consisting of hydrogen, halogen, methyl, ethyl, propyl, isopropyl, butyl, isobutyl, s-butyl, t-butyl, -OH, -SH, -NR₂₁R₂₂, trifluoromethyl, trifluoroethyl, methoxyl, ethoxyl, thiomethyl, thioethyl, -COH, -COOH, -COCH₃, -COOCH₃, -OC(O)CH₃, -SOH, -SO₂H, -SOCH₃, -SO₂CH₃, -OS(O)CH₃, and -C(O)NR₂₁R₂₂;
R₂₁ and R₂₂ are radicals, same or different, independently selected from the group consisting of hydrogen, and linear or branched (C₁-C₄)alkyl.

In still other preferred embodiment, the compound of formula (I) is one wherein:
R₁ is a radical selected from 2-furyl, 3-furyl, 2-thiophenyl, and 3-thiophenyl; being the radical optionally substituted by at least one radical selected from the group consisting of fluorine, chlorine, bromine, iodine, methyl, ethyl, propyl, isopropyl, trifluoromethyl, and -OH;
R₂ is a -NHR₉; being R₉ a radical selected from the group consisting of:
   methyl, ethyl, propyl, isopropyl, butyl, isobutyl, s-butyl, t-butyl, cyclopropyl, cyclobutyl, cyclopentyl, cyclohexyl, and phenyl; being the radical optionally substituted by at least one radical selected from the group consisting of halogen, -OH, -SH, nitro, cyano, amino, linear or branched (C₁-C₄)alkyl, halo-(C₁-C₆)alkyl, -O-(C₁-C₆)alkyl, -S-(C₁-C₆)alkyl, -COH, -COOH, -CO(C₁-C₄)alkyl, -COO(C₁-C₄)alkyl, -OC(O)(C₁-C₄)alkyl, -SOH, -SO₂H, -SO(C₁-C₄)alkyl, -SO₂(C₁-C₄)alkyl, -OS(O)(C₁-C₄)alkyl;
R₃ is a radical selected from the group consisting of -C≡C-CH₂CH₃, -C≡C-CH(CH₃)₂, -C≡C-CH₂CH₂OH, -C≡C-CH₂CH₂OH, -C≡C-CH₂COOH, -C≡C-CH₂CHO, -C≡C-CH₂CH₂CH₃, -C≡C-CH₂CH(CH₃)₂, -C≡C-CF₃, -C≡C-CH₂SH, -C≡C-CH₂NH₂, -C≡C-CH₂N(CH₃)₂, -C≡C-CH₂SOCH₃, -C≡C-CH₂SO₂CH₃, -C≡C-CH₂COOCH₃, (*E*)-CH=CH-CH₂CH₃, (*E*)-CH=CH-CH₂OH, (*E*)-CH=CH-CH₂SH, (*E*)-CH=CH-CH₂NH₂, (*E*)-CH=CH-CH(CH₃)₂, (*E*)-CH=CH-CHCH₃OH, (*E*)-CH=CH-CHCH₃SH, (*E*)-CH=CH-CHCH₃NH₂, (*E*)-CH=CH-C(CH₃)₃, (*E*)-CH=CH-C(CH₃)₂OH, (*E*)-CH=CH-C(CH₃)₂SH, (*E*)-CH=CH-C(CH₃)₂NH₂, (*E*)-CH=CH-CH₂COOH, (*E*)-CH=CH-CH₂COOC(CH₃)₃, (*E*)-CH=CH-CH₂N(CH₃)₂, (*E*)-CH=CH-CH₂COOCH₃, (*Z*)-CH=CH-CH₂CH₃, (*Z*)-CH=CH-CH₂OH, (*Z*)-CH=CH-CH₂SH, (*Z*)-CH=CH-CH₂NH₂, (*Z*)-CH=CH-CH(CH₃)₂, (*Z*)-CH=CH-CHCH₃OH, (*Z*)-CH=CH-CHCH₃SH, (*Z*)-CH=CH-CHCH₃NH₂, (*Z*)-CH=CH-C(CH₃)₃, (*Z*)-CH=CH-C(CH₃)₂OH, (*Z*)-CH=CH-C(CH₃)₂SH, (*Z*)-CH=CH-C(CH₃)₂NH₂, (*Z*)-CH=CH-CH₂COOH, (*Z*)-CH=CH-CH₂COOC(CH₃)₃, (*Z*)-CH=CH-CH₂N(CH₃)₂, and (*Z*)-CH=CH-CH₂COOCH₃.

Particularly preferred compounds of the present invention are:
(*E*)-4-(2-(Furan-2-yl)-4-(isopropylamino)quinazolin-6-yl)-2-methylbut-3-en-2-ol ; and
3-(2-(Furan-2-yl)-4-(isopropylamino)quinazolin-6-yl)prop-2-yn-1-ol.

In a further aspect, the present invention provides a process for the preparation of compounds of the formula (I), or its pharmaceutically acceptable salts, which process comprises the reaction of a anthranilic acid amide (II) with carbonic acid chloride followed by ring closure to obtain quinazolinones of general formula (III):

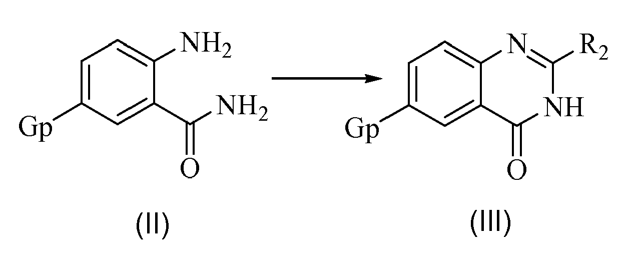

In formulae (II) and (III) Gp is a suitable leaving group; preferably Gp is an halogen (fluorine, chlorine, iodine, bromine); and R₂ is as defined above.

Compounds of formula (III) can be chlorinated in 4-position in order to introduce R₄ substitution in a following step to give (V): 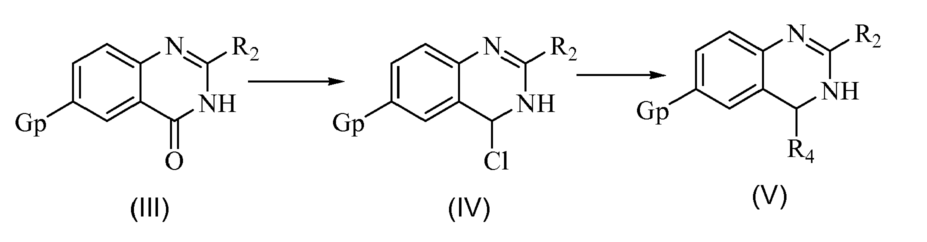 or alternatively, (III) can be converted to derivatives of formula (VI):

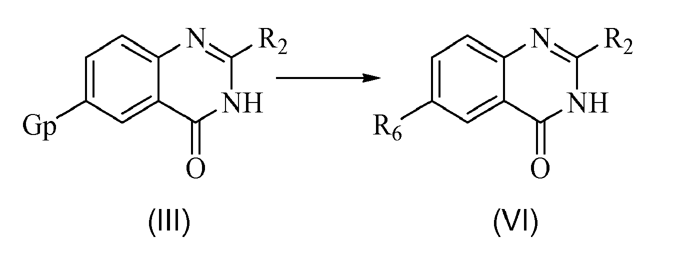

Compounds of formula (VI) can be chlorinated in 4-position in order to introduce R₄ substitution in a following step to give (VIII):

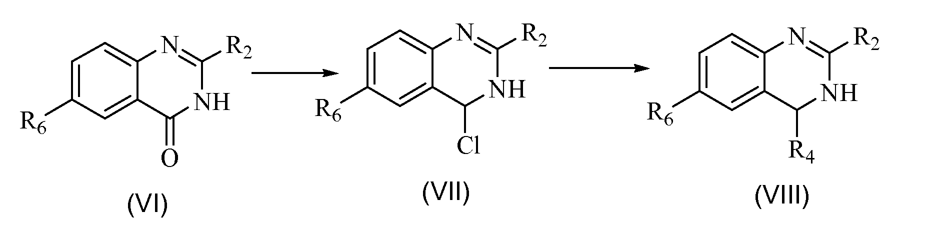

According to an embodiment of the present invention, compounds of formula (I) can be obtained as it is illustrated in Scheme I. 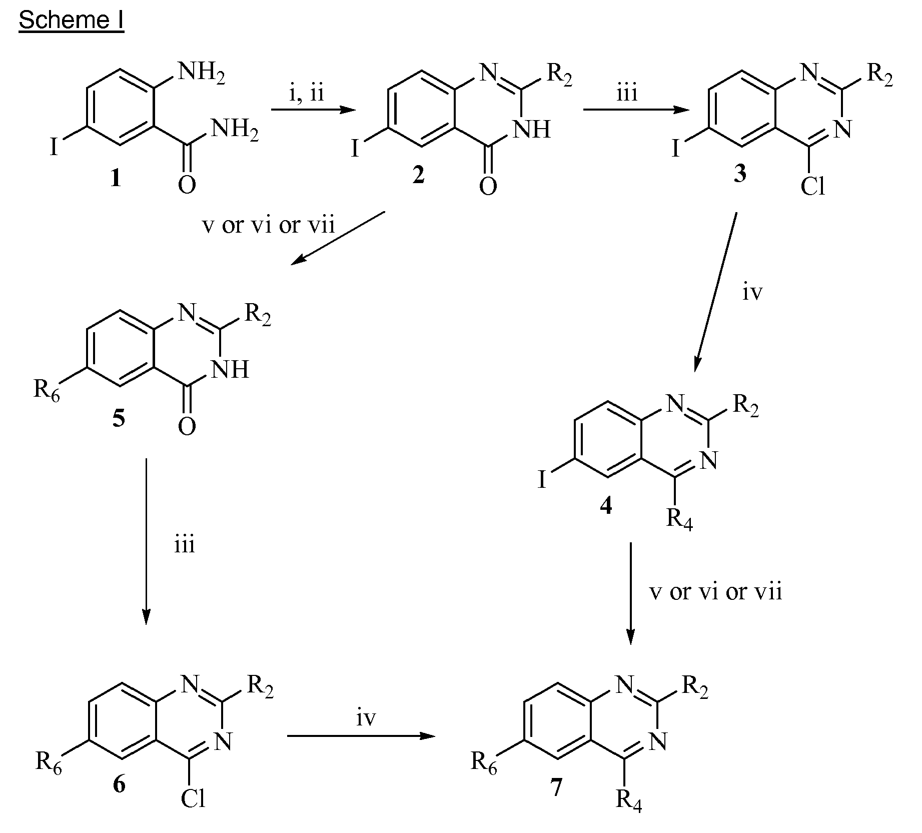 i) carbonic acid chloride, Et₃N, THF, rt; ii) NaOH, EtOH, reflux; iii) POCl₃, reflux or SOCl₂, DMF (cat.), reflux; iv) aliphatic amine, i-PrOH, 35 °C or aniline, HCl (cat.), i-PrOH, reflux; v) boronic acid, K₂CO₃ or Cs₂CO₃, Pd(PPh₃)₄ or Pd(OAc)₂ (cat.), dioxane/water 3:1, reflux; vi) alkene, Et₃N, Pd(OAc)₂ (cat.), (o-Tol)₃P (cat.), acetonitrile, 100 °C; vii) alkyne, Et₃N, Pd(PPh₃)₂Cl₂ (cat.), dppf (cat.), DMF, 50 °C.

The synthesis starts with anthranilic acid amide (1). The formation of an amide bond with different carbonic acid chlorides followed by ring closure under basic conditions leads to quinazolinones with general formula (2). They can be chlorinated in 4-position using phosphoryl trichloride or thionyl chloride with catalytic amounts of DMF to generate structures of the general formula (3) which are converted to different 4-amino or 4-anilinoquinazolines of general structure (4) by established aromatic substitution reactions.

Different well-known Palladium-catalyzed cross-couplings like the Suzuki-coupling, the Heck reaction or the Sonogashira-reaction furnish the final structures of general formula (7). On the other hand, the quinazolinones (2) can be converted to derivatives with general structure (5) by the described Palladium chemistry. They are further converted to the 4-chloro-derivatives with the described methods to obtain compounds of general structure (6). They are converted to compounds of the final structure (7) by using aromatic substitution reactions in the last step of the synthesis route.

Alternatively, the compounds of formula (I) can be obtained as it is illustrated in Scheme 2 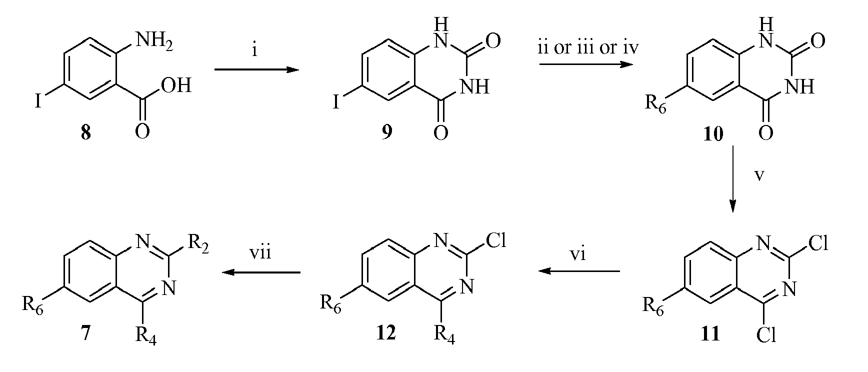
i) CO(NH₂)₂, 200 °C; ii) boronic acid, K₂CO₃ or Cs₂CO₃, Pd(PPh₃)₄ or Pd(OAc)₂ (cat.), dioxane/water 3:1, reflux; iii) alkene, Et₃N, Pd(OAc)₂ (cat.), (o-Tol)₃P (cat.), acetonitrile, 100 °C; iv) alkyne, Et₃N, Pd(PPh₃)₂Cl₂ (cat.), dppf (cat.), DMF, 50 °C; v) POCl₃, Me₂NPh, reflux; vi) aliphatic amine, THF, rt or aniline, EtOH, rt; vii) boronic acid, K₂CO₃, (PPh₃)₂PdCl₂, DME/H₂O/EtOH, 120 °C

The synthesis of the described molecules starts from 2-amino-5-iodo benzoic acid (8) (J. Med. Chem. 2007, 50, 2297-2300). It is converted with urea to obtain the quinazolin-2,4-dione (9). By the described methods of palladium chemistry this can be converted to the 6-substituted quinazolinones of general structure (10). Chlorination of the 2- and the 4-position in one step occurs by reflux in phosphoryl trichloride and dimethylaniline to obtain molecules with the general structure (11) (J. Med. Chem. 2007, 50, 2297-2300). The selective amination of the 4-position leading to (12) is realized with aliphatic amines in THF or anilines in ethanol (J. Med. Chem. 1995, 38, 2763-2773; Bioorg. Med. Chem. 2006, 14, 3307-3319). The Suzuki-coupling leads to the final structures of general formula (7). This synthesis route is more efficient for the synthesis of derivatives with a variation in 2-position (Tetrahedron Lett. 2006, 47, 8251-8254).

It should be noted that the general procedures are shown as it relates to preparation of compounds having unspecified stereochemistry. However, such procedures are generally applicable to those compounds of a specific stereochemistry, e.g., where the stereochemistry at a sterogenic center is (S) or (R). In addition, the compounds having one stereochemistry (e.g., (R)) can often be utilized to produce those having opposite stereochemistry (i.e., (S)) using well-known methods, for example, by inversion. The same applies for Z / E enantiomers.

The term "pharmaceutically acceptable salt", as used herein, refers to salts derived from organic and inorganic acids. The compound of the general formula (I) may be converted into its pharmaceutically acceptable salts, or its pharmaceutically acceptable solvates by conventional methods. For example, such salts may be prepared by treating one or more of the compounds with an aqueous solution of the desired pharmaceutically acceptable metallic hydroxide or other metallic base and evaporating the resulting solution to dryness, preferably under reduced pressure in a nitrogen atmosphere. Alternatively, a solution of the compound of formula (I) may be mixed with an alkoxide of the desired metal, and the solution subsequently evaporated to dryness. The pharmaceutically acceptable hydroxides, bases, and alloxides include those worth cations for this purpose, including (but not limited to), potassium, sodium, ammonium, calcium, and magnesium. Other representative pharmaceutically acceptable salts include hydrochloride, hydrobromide, sulphate, bisulphate, lactate, phosphate, tosylate, citrate, maleate, fumarate, succinate, tartrate, acetate, oxalate, propionate, nitrate, methanesulfonate, benzoate and similarly known acceptable acids.

The term "(C₁-C₄)alkyl" as used herein refers to a saturated branched or linear hydrocarbon chain with 1 to 4 hydrocarbon atoms. Preferably "(C₁-C₄)alkyl" is an unsubstituted group selected from methyl, ethyl, propyl, isopropyl, butyl, isobutyl, s-butyl and t-butyl.

The term "(C₁-C₆)-alkoxy" as used herein refers to a saturated branched or linear hydrocarbon chain with 1 to 6 hydrocarbon atoms (i.e. (C₁-C₆)alkyl groups as defined above) linked to an oxygen, thus (C₁-C₆)alkyl-O. Preferably "(C₁-C₆)-alkoxy" is an unsubstituted group selected from methoxy, ethoxy, propoxy, isopropoxy, butoxy, isobutoxy, s-butoxy, and t-butoxy.

The term "halogen" is meant to include fluorine, chlorine, bromine and iodine.

It is clear to a person skilled in the art that certain compounds of the present invention possess asymmetric carbon atoms (optical centers) and/or double bonds; the racemates, enantiomers, diastereomers, geometric isomers and individual isomers are all intended to be encompassed within the scope of the present invention.

Certain compounds of the present invention can exist in unsolvated forms as well as in solvated forms, including hydrated forms, and are intended to be encompassed within the scope of the present invention. Certain compounds of the present invention may exist in multiple crystalline or amorphous forms. In general, all physical forms are equivalent for the uses contemplated by the present invention and are intended to be within the scope of the present invention.

The pharmaceutical composition (e. g. formulation) may comprise a therapeutically effective amount of the compound of formula (I), as defined above, together with one or more pharmaceutically acceptable excipients or carriers such as adjuvants, diluents, fillers, buffers, stabilisers, preservatives, lubricants.

The term "pharmaceutically acceptable" as used herein pertains to compounds, materials, compositions, and/or dosage forms which are, within the scope of sound medical judgment, suitable for use in contact with the tissues of a subject (e. g. human) without excessive toxicity, irritation, allergic response, or other problem or complication, commensurate with a reasonable benefit/risk ratio. Each carrier, excipient, etc. must also be "acceptable" in the sense of being compatible with the other ingredients of the formulation.

Suitable carriers, excipients, etc. can be found in standard pharmaceutical texts, for example, Remington's Pharmaceutical Sciences, 18th edition, Mack Publishing Company, Easton, Pa., 1990.

The term "therapeutically effective amount," as used herein, pertains to that amount of an active compound, or a material, composition or dosage form comprising an active compound, which is effective for producing some desired therapeutic effect.

In addition to salt forms, the present invention provides compounds which are in a prodrug form. Prodrugs of the compounds described herein are those compounds that readily undergo chemical changes under physiological conditions to provide a compound of formula (I). A prodrug is a pharmacologically active or inactive compound that is modified chemically through in vivo physiological action, such as hydrolysis, metabolism and the like, into a compound of this invention following administration of the prodrug to a patient. Additionally, prodrugs can be converted to the compounds of the present invention by chemical or biochemical methods in an ex vivo environment. For example, prodrugs can be slowly converted to the compounds of the present invention when placed in a transdermal patch reservoir with a suitable enzyme. The suitability and techniques involved in making and using prodrugs are well known by those skilled in the art. Examples of a masked carboxylate anion include a variety of esters, such as alkyl (for example, methyl, ethyl), cycloalkyl (for example, cyclohexyl), arylalkyl (for example, benzyl, p-methoxybenzyl), and alkylcarbonyloxyalkyl (for example, pivaloyloxymethyl). Amines have been masked as arylcarbonyloxymethyl substituted derivatives which are cleaved by esterases in vivo releasing the free drug and formaldehyde. Also, drugs containing an acidic NH group, such as imidazole, imide, indole and the like, have been masked with N-acyloxymethyl groups. Hydroxyl groups have been masked as esters and ethers.

As it is shown below, the compounds of the present invention are Aurora A inhibitors, being useful in the treatment of a disease mediated by Aurora A.

In a preferred embodiment it is provided the use of a compound of formula (I) as defined in the preferred embodiments pointed out above for the manufacture of a medicament for the treatment of a disease mediated by Aurora A kinase. This aspect of the invention can also be formulated as compound of formula (I) for use in the treatment of a disease mediated by Aurora A kinase.

The term "a disease mediated by Aurora A kinase", as used herein pertains to a condition in which Aurora A and/or the action of Aurora A is important or necessary, e.g., for the onset, progress, expression, etc. of that condition.

Since aurora kinases are known to have a central role in the cell cycle and in particular Aurora A kinase shows an elevated expression in more than 50% of colorectal, ovarian and gastric cancers and in more than 95% of invasive adenocarcinomas in a preferred embodiment of the present invention the diseases, conditions and/or disorders, which can be prevented, ameliorated or treated with the compounds of the present invention are hyperproliferative diseases. A disease is considered to benefit from reduced aurora kinase, in particular Aurora A kinase activity, if a reduction of Aurora kinase activity of at least 10%, preferably of at least 20%, preferably of at least 30%, leads to an improvement of at least one clinical indicator of that disease. Examples of such indicators are proliferation rate, which is preferably reduced, cellular differentiation, which is preferably induced etc.

It is further preferred that the hyperproliferative diseases are selected from the group consisting of precancerosis; dysplasia; metaplasia; carcinomas of the gastrointestinal or colorectal tract, liver, pancreas, kidney, bladder, prostate, endometrium, ovary, testes, melanoma, dysplastic oral mucosa, invasive oral cancers, small cell and non-small cell lung carcinomas, hormone-dependent breast cancers, hormone-independent breast cancers, transitional and squamous cell cancers, neurological malignancies including neuroblastoma, gliomas, astrocytomas, osteosarcomas, soft tissue sarcomas, hemangioamas, endocrinological tumors, hematologic neoplasias including leukemias, lymphomas, and other myeloproliferative and lymphoproliferative diseases, carcinomas in situ, hyperplastic lesions, adenomas, fibromas, histiocytosis, chronic inflammatory proliferative diseases, vascular proliferative diseases and virus-induced proliferative diseases, skin diseases characterized by hyperproliferation of keratinocytes and/or T cells. Particular preferred diseases treatable with the compounds of the present invention are colorectal, ovarian, prostatic and gastric cancers and adenocarcinomas, more preferably invasive adenocarcinomas.

The precancerosis treatable with the compounds of the present invention are preferably selected from the group consisting of precancerosis of the skin, in particular actinic keratosis, cutaneaous horn, actinic cheilitis, tar keratosis, arsenic keratosis, x-ray keratosis, Bowen's disease, bowenoid papulosis, lentigo maligna, lichen sclerosus, and lichen rubber mucosae; precancerosis of the digestive tract, in particular erythroplakia, leukoplakia, Barrett's esophagus, Plummer-Vinson syndrome, crural ulcer, gastropathia hypertrophica gigantea, borderline carcinoma, neoplastic intestinal polyp, rectal polyp, porcelain gallbladder; gynaecological precancerosis, in particular carcinoma ductale in situ (CDIS), cervical intraepithelial neoplasia (CIN), leukoplakia, endometrial hyperplasia (grade III), vulvar dystrophy, vulvar intraepithelial neoplasia (VIN), hydatidiform mole; urologic precancerosis, in particular bladder papillomatosis, Queyrat's erythroplasia, testicular intraepithelial neoplasia (TIN), leukoplakia; carcinoma *in situ* (CIS); precancerosis caused by chronic inflammation, in particular pyoderma, osteomyelitis, acne conglobata, lupus vulgaris, and fistula.

Dysplasia is frequently a precursor of cancer, and is found mainly in the epithelia; it is the most disorderly form of non-neoplastic cell growth, involving a loss in individual cell uniformity and in the architectural orientation of cells. Dysplastic cells often have abnormally large, deeply stained nuclei, and exhibit pleomorphism. Dysplasia characteristically occurs where there exists chronic irritation or inflammation. Dysplastic disorders which can be treated with the compounds of the present invention include, but are not limited to, anhidrotic ectodermal dysplasia, anterofacial dysplasia, asphyxiating thoracic dysplasia, atriodigital dysplasia, bronchopulmonary dysplasia, cerebral dysplasia, cervical dysplasia, chondroectodermal dysplasia, cleidocranial dysplasia, congenital ectodermal dysplasia, craniodiaphysial dysplasia, craniocarpotarsal dysplasia, craniometaphysial dysplasia, dentin dysplasia, diaphysial dysplasia, ectodermal dysplasia, enamel dysplasia, encephalo-ophthalmic dysplasia, dysplasia epiphysialis heminelia, dysplasia epiphysialis multiplex, dysplasia epiphysalis punctata, epithelial dysplasia, faciodigitogenital dysplasia, familial fibrous dysplasia of jaws, familial white folded dysplasia, fibromuscular dysplasia, fibrous dysplasia of bone, florid osseous dysplasia, hereditary renal-retinal dysplasia hidrotic ectodermal dysplasia, hypohidrotic ectodermal dysplasia, lymphopenic thymic dysplasia, mammary dysplasia, mandibulofacial dysplasia, metaphysical dysplasia, Mondini dysplasia, monostotic fibrous dysplasia, mucoepithelial dysplasia, multiple epiphysial dysplasia, oculoauriculovertebral dysplasia, oculodentodigital dysplasia, oculovertebral dysplasia, odontogenic dysplasia, ophthalmomandibulomelic dysplasia, periapical cemental dysplasia, polyostotic fibrous dysplasia, pseudoachondroplastic spondyloepiphysial dysplasia, retinal dysplasia, septo-optic dysplasia, spondyloepiphysial dysplasia, and ventriculoradial dysplasia. Metaplasia is a form of controlled cell growth in which one type of adult or fully differentiated cell substitutes for another type of adult cell. Metaplastic disorders, which are treatable are preferably selected from the group consisting of agnogenic myeloid metaplasia, apocrine metaplasia, atypical metaplasia, autoparenchymatous metaplasia, connective tissue metaplasia, epithelial metaplasia, intestinal metaplasia, metaplastic anemia, metaplastic ossification, metaplastic polyps, myeloid metaplasia, primary myeloid metaplasia, secondary myeloid metaplasia, squamous metaplasia, squamous metaplasia of amnion, symptomatic myeloid metaplasia and regenerative metaplasia.

Many skin diseases are characterized by hyperproliferation of keratinocytes and/or T cells. Examples of such diseases which are treatable with the compounds of the present invention comprise without limitations psoriasis in particular psoriasis vulgaris, psoriasis capitis, psoriasis guttata, psoriasis inversa; neurodermatitis; ichtyosises; alopecia areata; alopecia totalis; alopecia subtotalis; alopecia universalis; alopecia diffusa; atopic dermatitis; lupus erythematodes of the skin; dermatomyositis of the skin; atopic eczema; morphea; scleroderma; alopecia areata Ophiasis type; androgenic alopecia; allergic contact dermatitis; irritative contact dermatitis; contact dermatitis; pemphigus vulgaris; pemphigus foliaceus; pemphigus vegetans; scarring mucous membrane pemphigoid; bullous pemphigoid; mucous membrane pemphigoid; dermatitis; dermatitis herpetiformis Duhring; urticaria; necrobiosis lipoidica; erythema nodosum; prurigo simplex; prurigo nodularis; prurigo acuta; linear IgA dermatosis; polymorphic light dermatosis; erythema Solaris; exanthema of the skin; drug exanthema; purpura chronica progressiva; dihydrotic eczema; eczema; fixed drug exanthema; photoallergy skin reaction; and periorale dermatitis.

It is suspected that Aurora kinases are also involved in mediating activation of estrogen receptor (ER) controlled genes. Thus, in a further preferred embodiment the hyperproliferative disorders which are treatable by the Aurora kinase inhibitors of the present invention are those which benefit from a reduced estrogen receptor signalling. It is known that an increased activity of genes controlled by estrogen receptor is responsible or contributes to various hyperproliferative diseases. Thus preferred diseases, conditions and/or disorders which can be treated with the compounds of the present invention are selected from the group consisting of mammary tumors, endometrial tumors and tumors of the uterus. The ability of the compounds of the present invention to inhibit estrogen receptor signalling can be confirmed with any of a number of art known methods involving, e.g. the transfection of cells with plasmids comprising ER recognition sequences upstream of reporter genes, e.g. CAT, luciferase etc. Similarly, someone of skill in the art is capable of assessing whether a given hyperproliferative disease involves increased estrogen receptor signalling by determining the expression level of an ER controlled gene, e.g. cathepsin D, lactoferrin, or IGF-I etc.

The above data suggest that strong and selective inhibition of Aurora A activity makes sense for the treatment of tumors. Therefore the compounds of formula (I) herein described have a potential use as chemotherapeutical agents. The application to patients with tumor diseases related to Aurora A overexpression would inhibit this kinase and could lead to a deceleration or a stop of the tumor growth, what could lead to increased healing chances or better life quality for the patients.

Thus, the present invention also provides active compounds which are antiproliferative agents. The term "antiproliferative agent" as used herein, refers to a compound which treats a proliferative condition (i.e., a compound which is useful in the treatment of a proliferative condition).

The terms "cell proliferation", "proliferative condition", "proliferative disorder", and "proliferative disease", are used interchangeably herein and pertain to an unwanted or uncontrolled cellular proliferation of excessive or abnormal cells which is undesired, such as, neoplastic or hyperplastic growth, whether in vitro or in vivo. Examples of proliferative conditions include, but are not limited to, benign, pre-malignant, and malignant cellular proliferation, including but not limited to, neoplasms and tumors (e.g., histocytoma, glioma, astrocyoma, osteoma), cancers (e.g., lung cancer, small cell lung cancer, gastrointestinal cancer, bowel cancer, colon cancer, breast carcinoma, ovarian carcinoma, prostate cancer, testicular cancer, liver cancer, kidney cancer, bladder cancer, pancreas cancer, brain cancer, sarcoma, osteosarcoma, Kaposi's sarcoma, melanoma), leukemias, psoriasis, bone diseases, fibroproliferative disorders (e.g., of connective tissues), and atherosclerosis.

The term "treatment," as used herein in the context of treating a condition, pertains generally to treatment and therapy, whether of a human or an animal (e.g., in veterinary applications), in which some desired therapeutic effect is achieved, for example, the inhibition of the progress of the condition, and includes a reduction in the rate of progress, a halt in the rate of progress, amelioration of the condition, and cure of the condition. Treatment as a prophylactic measure (e.g., prophylaxis) is also included. The term "treatment" includes combination treatments and therapies, in which two or more treatments or therapies are combined, for example, sequentially or simultaneously. Examples of treatments and therapies include, but are not limited to, chemotherapy (the administration of active agents, including, e.g., drugs, antibodies (e.g., as in immunotherapy), prodrugs (e.g., as in photodynamic therapy, GDEPT, ADEPT, etc.); surgery; radiation therapy; and gene therapy.

The active compound or pharmaceutical composition comprising the active compound may be administered to a subject by any convenient route of administration, whether systemically/ peripherally or at the site of desired action, including but not limited to, oral (e.g. by ingestion); topical (including e.g. transdermal, intranasal, ocular, buccal, and sublingual); pulmonary (e.g. by inhalation or insufflation therapy using, e.g. an aerosol, e.g. through mouth or nose); rectal; vaginal; parenteral, for example, by injection, including subcutaneous, intradermal, intramuscular, intravenous, intraarterial, intracardiac, intrathecal, intraspinal, intracapsular, subcapsular, intraorbital, intraperitoneal, intratracheal, subcuticular, intraarticular, subarachnoid, and intrasternal by implant of a depot, for example, subcutaneously or intramuscularly.

The subject may be a eukaryote, an animal, a vertebrate animal, a mammal, a rodent (e.g. a guinea pig, a hamster, a rat, a mouse), murine (e.g. a mouse), canine (e.g. a dog), feline (e.g. a cat), equine (e.g. a horse), a primate, simian (e.g. a monkey or ape), a monkey (e.g. marmoset, baboon), an ape (e.g. gorilla, chimpanzee, orangutang, gibbon), or a human. Furthermore, the present invention covers all possible combinations of particular and preferred groups described hereinabove.

Throughout the description and claims the word "comprise" and variations of the word, such as "comprising", are not intended to exclude other technical features, additives, components, or steps.

Furthermore, the present invention covers all possible combinations of particular and preferred groups described hereinabove.

Additional objects, advantages and features of the invention will become apparent to those skilled in the art upon examination of the description or may be learned by practice of the invention. The following examples and drawings are provided by way of illustration, and are not intended to be limiting the present invention.

### BRIEF DESCRIPTION OF THE DRAWINGS

Figure 1. DNA profiles of HeLa cells treated with DMSO (Control) or 2 µM ZM1 or increasing concentrations of compounds (A) and (C) for 24 h were evaluated by flow cytometry. 2n, 4n and 8n reflect relative DNA content.
Figure 2. Effect of the different compounds on histone H3 phosphorylation on Ser 10. HeLa cells were treated with ZM1 or the different compounds of the invention and synchronized in mitosis with nocodazole. Histone H3 phosphorylation degree (p-HH3) was determined by Western Blot analysis. AurA and α-tubulin (αTub) levels were used as synchronization and loading controls.
Figure 3. Images from time-lapse analysis of cells treated with DMSO, ZM1, (A) and (C). HeLa cells expressing GFP-Histone 2B (second rows) and mRFP-α-tubulin were incubated in medium containing DMSO (Control), 2 µM of ZM1 or 40 µM of (A) or (C). Images were captured for a period of 16 h-24 h using a time-lapse imaging system. In each panel the upper row shows the phase contrast images and the lower row the GFP signal. Bar, 10 µm.
Figure 4. Quantification of the phenotypes observed during the time-lapse recording of cells treated with the different compounds.
   A- Bar graph showing the mean percentage of non dividing cells dying over the period of the time-lapse recording. The values obtained from more than 6 fields from two independent experiments were averaged.
   B- Average percentage of cells entering mitosis per hour of observation. The percentage of cells entering mitosis in each filmed field was divided by the number of filming hours. The values obtained from more than 6 fields from two independent experiments were averaged.
   C- Outcome of cells entering mitosis. Cells entering mitosis were followed carefully over the time and each mitotic event was classified in one of the following four categories: normal division (cell division), return to interphase without chromosome segregation and without dividing (no division), cytokinesis failure (division errors), and cell death without resolving mitosis (cell death).
   D- Box plots measuring time spent in mitosis in the presence of 2 µM ZM1 or 40 µM (A) or (C). Values obtained from more than 6 fields from two independent experiments were averaged.

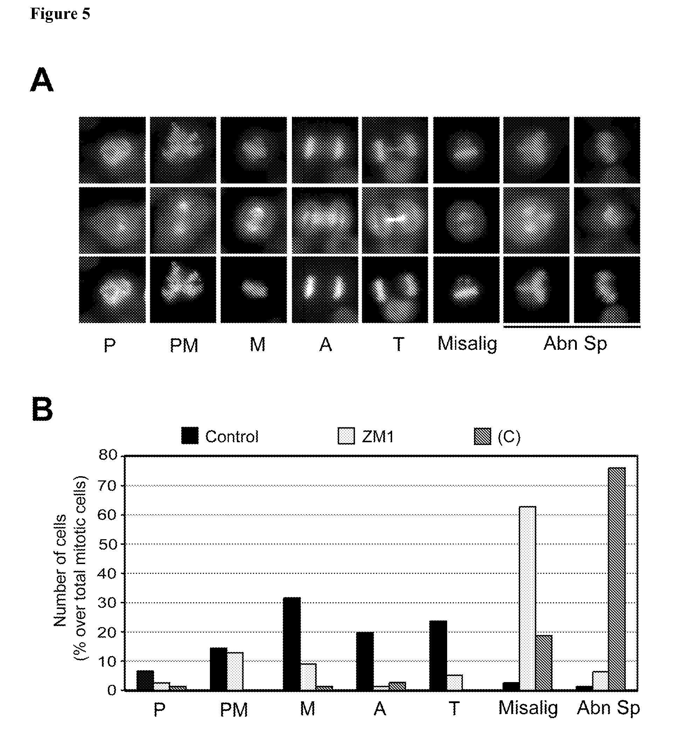

Figure 5. Mitotic phenotypes of cells incubated in the presence of the different compounds.
   A- HeLa cells expressing constitutively GFP-Histone H2B and mRed fluorescent protein-conjugated α-tubulin were incubated for 24 h in the presence of DMSO or 2 µM ZM1 or 40 µM (C), fixed and examined. Mitotic cells were scored and classified according to the different categories shown. Representative images for the different categories are shown: prophase (P), prometaphase (PM), metaphase (M), anaphase (A), telophase (T), metaphase spindles with lagging chromosomes (Misalign) and abnormal spindles, defined as mitotic structures whose MT are not organized in a canonical bipolar spindle (Abn S). MT are in red and DNA is in green.
   B- Quantification of the number of cells in the different categories defined in A. Untreated cells (black bars), cells treated with ZM1 (white bars) and (C) (grey bars).

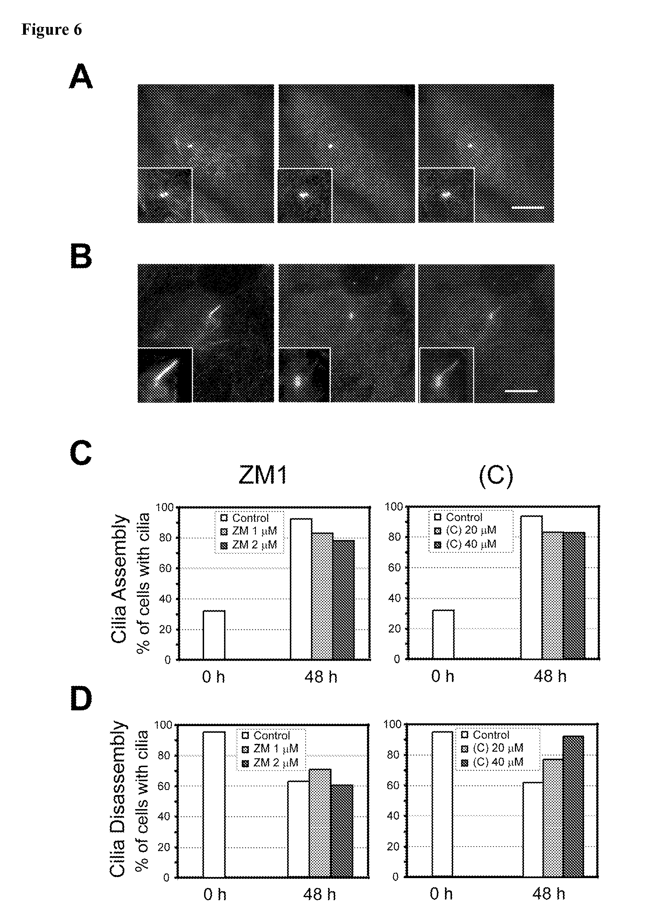

Figure 6. Compound (C) blocks cilia disassembly.
   A- Representative immunofluorescence images of interphase RPE cells. The anti-acetylated tubulin and anti-α-tubulin antibody decorate the centrosome of actively growing RPE cells
   B- Serum starved RPE cells develop a cilia from one of the two centrioles. The anti acetylated tubulin antibody decorates both centrioles including the basal body and its connected cilium. Acetylated tubulin is in red, α-tubulin in green and the DNA in blue in the merge images.
   C- Quantification of the number of cells forming a cilium when serum starved in the presence of the compounds. No differences are observed among cells incubated with the different compounds. Graphs show a representative experiment repeated 3 times.
   D- Quantification of the number of cells loosing cilia when they are transferred to a medium containing serum and the compounds indicated after serum starvation. Cilia disassembly is impaired in the presence of compound (C). Graphs show a representative experiment repeated three times.

### EXAMPLES

### Example 1. Preparation of compounds

All reagents were commercially obtained from Acros, Aldrich, Alfa Aesar and Fluka and used without further purification. Melting points were measured with a Boetius-micro hot stage and are uncorrected. ¹H and ¹³C NMR spectra were recorded on a Varian Gemini 200 (200 MHz for ¹H NMR; 50 MHz for ¹³C), a Varian Gemini 300 (300 MHz for ¹H NMR; 75 MHz for ¹³C NMR) and Bruker Advance-DRX 400 (400 MHz for ¹H NMR; 100 MHz for ¹³C NMR); the residual solvent peak was used as an internal reference. HRMS were obtained on a Bruker Daltonics APEX II (for ESI) and on a Meß- und Analysentechnik Bremen MAT8035 (EI). Reactions involving moisture-sensitive reactants were performed in flame-dried glassware under an argon atmosphere; reactants were added via syringe. Flash column chromatography was performed on silica gel (Acros 60 A, 0.035-0.070 mm) and analytical TLC on pre-coated silica gel plates (Merck 60 F254, 0.25 mm).

### 2-Amino-5-iodobenzamide (2)

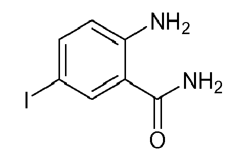

To a stirred solution of 2-aminobenzamide (5.72 g, 42.0 mmol) and NaHCO₃ (3.52 g, 42.0 mmol) in water (1.3 I) powdered iodine (11.7 g, 46.2 mmol) was added portionwise in 1 h. The solution was stirred overnight at rt. Afterwards NaHSO₃ (0.87 g, 8.40 mmol) was added. The solution was extracted with ethyl acetate (3 x 800 ml). After drying with Na₂SO₄ the organic phase was removed under reduced pressure. The crude product was recrystallized with water/methanol-mixture (10:1, v/v, 100 ml) yielding 2 (9.07 g, 34.6 mmol, 82%) as white crystals; mp. 197-198 °C. ¹H-NMR (300 MHz, DMSO-d₆): δ = 6.57 (d, J = 8.7 Hz, 1 H, arom.), 6.74 (s, 2 H, NH₂), 7.19 (s, 2 H, CONH₂), 7.42 (dd, J = 8.7 Hz, J = 1.8 Hz, 1 H, arom.), 7.84 (d, J = 2.1 Hz, 1 H, arom.). ¹³C-NMR (75 MHz, DMSO-d₆): δ = 74.5, 116.1, 119.0, 136.5, 139.9, 149.8, 169.9. HR EL-MS: calcd. for (C₇H₇IN₂O): 261.96032, found: 261.95953

### N-(2-Carbamoyl-4-iodophenyl)furan-2-carboxamide (2a)

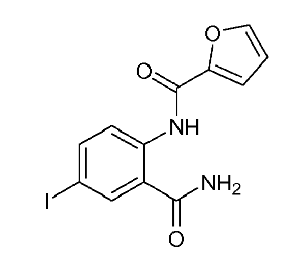

To a solution of 2-Amino-5-iodobenzamide (2) (2.06 g, 7.86 mmol) in THF (23 ml) triethylamine (2.19 ml, 15.7 mmol) and furan-2-carbonyl chloride (0.77 ml, 7.86 mmol) were added by a syringe. After 2 h (TLC-control) the solvent was removed under reduced pressure. The residual solid was washed with cold ethanol yielding pure (2a) (2.76 g, 7.75 mmol, 99%) as a white solid; mp.: 218-220 °C. ¹H-NMR (300 MHz, DMSO-d₆): δ = 6.75 (dd, J = 3.0 Hz, J = 3.0 Hz, 1 H, arom.), 7.26 (d, J = 3.0 Hz, 1 H, arom.), 7.90 (dd, J = 8.7 Hz, J = 1.8 Hz, 1 H, arom.), 7.99 (s, 1 H, arom.), 8.22 (d, J = 1.8 Hz, 1 H, arom.), 8.46 (d, J = 9.0 Hz, 1 H, arom.), 8.49 (s, 2 H, NH₂), 12.72 (s, 1 H, NH-CO). ¹³C-NMR (75 MHz, DMSO-d₆): δ = 87.2, 113.4, 116.1, 122.2, 122.8, 137.5, 139.8, 141.6, 146.9, 148.2, 156.4, 170.2. HR ESI-MS: (M + Na)⁺ calcd. for C₁₂H₉IN₂O₃Na: 378.95555, found: 378.95501.

### 2-(Furan-2-yl)-6-iodoguinazolin-4(3H)-one (5)

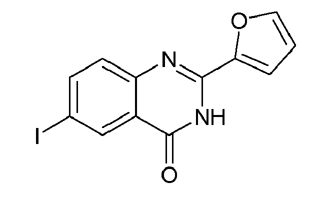

A solution of N-(2-carbamoyl-4-iodophenyl)furan-2-carboxamide (2a) (2.76 g, 7.75 mmol) in 5% NaOH (39 ml) and ethanol (20 ml) was refluxed for 5 min. After cooling down the solution to 0 °C a voluminous white precipitate fell out and was filtered of. It was dried yielding pure 5 (2.05 g, 6.06 mmol, 78%) as a white solid; mp.: 222-223 °C. ¹H-NMR (300 MHz, DMSO-d₆): δ = 6.75 (dd, J = 3.6 Hz, J = 3.3 Hz, 1 H, arom.), 7.26 (d, J = 2.7 Hz, 1 H, arom.), 7.91 (dd, J = 8.7 Hz, J = 2.1 Hz, 1 H, arom.), 8.00 (s, 1 H, arom.), 8.21 (d, J = 2.4 Hz, 1 H, arom.), 8.45 (d, J = 9.3 Hz, 1 H, arom.), 8.49 (s, 1 H, NH). ¹³C-NMR (75 MHz, DMSO-d₆): δ = 87.2, 113.4, 116.1, 122.2, 122.8, 137.5, 139.8, 141.6, 146.9, 148.2, 156.4, 170.2. HR ESI-MS: (M + H)⁺ calcd. for C₁₂H₈IN₂O₂: 338.96305, found: 338.96250.

### 4-Chloro-2-(furan-2-yl)-6-iodoguinazoline (7)

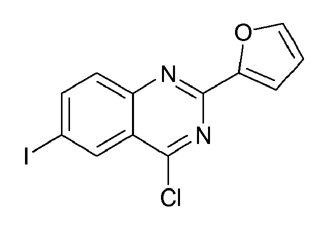

A solution of 2-(furan-2-yl)-6-iodoquinazolin-4(3H)-one (5) (1.95 g, 5.76 mmol) in phosphoryl trichloride (50 ml) was refluxed for 3 h (TLC-control). The white suspension became a clear solution. The excess of phosphoryl trichloride was removed under reduced pressure. The remaining solid was suspended in ethyl acetate (150 ml) and washed with 1 M NaOH (5 x 100 ml) solution. The combined aqueous phases were reextracted with ethyl acetate (100 ml). The combined organic phases were washed with brine (150 ml) and dried over Na₂SO₄. After removal of the solvent under reduced pressure the crude product was purified by column chromatography (n-hexane/ethyl acetate 5:1 v/v, R_{f}: 0.31) yielding 7 (1.77 g, 4.97 mmol, 86%) as a yellow solid, mp.: 183-184 °C. ¹H-NMR (300 MHz, DMSO-d₆): δ = 6.79 (d, J = 3.6 Hz, 1 H, arom.), 7.50 (d, J = 8.7 Hz, 1 H, arom.), δ = 7.69 (d, J = 3.6 Hz, 1 H, arom.), 8.05 (s, 1 H, arom.), 8.11 (dd, J = 8.9 Hz, J = 2.3 Hz, 1 H, arom.), 8.39 (d, J = 2.1 Hz, 1 H, arom.). ¹³C-NMR (50 MHz, DMSO-d₆): δ = 91.5, 112.7, 115.5, 122.8, 128.9, 134.2, 143.0, 144.5, 145.5, 147.1, 160.1, 150.0. HR ESI-MS: (M + H)⁺ calcd. for C₁₂H₇ClIN₂O: 356.92916, found: 356.92861.

### 2-(Furan-2-yl)-6-iodo-N-isopropylquinazolin-4-amine (10)

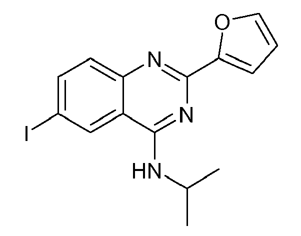

A solution of 4-chloro-2-(furan-2-yl)-6-iodoquinazoline (7) (1.70 g, 4.70 mmol) and i-propylamine (1.02 ml, 11.9 mmol) in i-propanol (70 ml) was stirred overnight at 35 °C. The yellow suspension turned to a clear solution. After removal of the solvent under reduced pressure the crude product was purified by column chromatography (n-hexane/ethyl acetate 3:1 v/v, R_{f}: 0.35) yielding 10 (1.77 g, 4.67 mmol, 98%) as a orange-coloured solid, mp.: 205 °C. ¹H-NMR: (200 MHz, DMSO-d₆): δ [ppm] = 1.31 (d, J = 6.6 Hz, 6 H, CH(CH₃)₂), 4.61 (se, J = 6.8 Hz, 1 H, CH(CH₃)₂) 6.67 (dd, J = 3.4 Hz, J = 3.3 Hz, 1 H, arom.), 7.25 (d, J = 2.5 Hz, 1 H, arom.), 7.48 (d, J = 8.8 Hz, 1 H, arom.), 7.89 (d, J = 2.6 Hz, 1 H, arom.), 8.01 (dd, J = 8.8 Hz, J = 1.9 Hz, 1 H, arom.), 8.09 (d, J = 7.6 Hz, 1 H, NH), 8.75 (d, J = 1.8 Hz, 1 H, arom.). ¹³C-NMR (50 MHz, DMSO-d₆): δ = 21.9 (2 C), 42.2, 89.9, 112.1, 112.9, 115.6, 129.5, 131.4, 141.0, 145.0, 148.9, 153.0, 153.6, 157.5. HR ESI-MS: (M + H)⁺ calcd. for C₁₅H₁₅IN₃O: 380.02598, found: 380.02529.

### (E)-4-(2-(Furan-2-yl)-4-(isopropylamino)quinazolin-6-yl)-2-methylbut-3-en-2-ol (A)

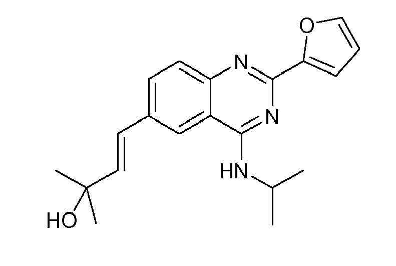

In a Pyrex^{®}-pressure tube a solution of 6-(3-aminophenyl)-2-(furan-2-yl)-N-isopropylquinazolin-4-amine (10) (85 mg, 0.22 mmol), 2-methylbut-3-en-2-ol (70 µl, 0.67 mmol), tri-o-tolylphosphine (6.7 mg, 22.0 µmol), triethylamine (94 µl, 0.67 mmol) and palladium acetate (2.5 mg, 11.0 µmol) in acetonitrile (1.0 ml) was heated to 100 °C. The yellow suspension turned to a black solution in 20 min. After 48 h the solvent was removed under reduced pressure and the crude product was purified by column chromatography (n-hexane/ethyl acetate 1:3 v/v, R_{f}: 0.38) yielding compound (A) (65 mg, 0.19 mmol, 86%) as a yellow solid, mp.: 144-146 °C. ¹H-NMR (200 MHz, CD₃OD): δ = 1.39 (d, J = 7.4 Hz, 6 H, CH(CH₃)₂), 1.40 (s, 6 H, C(CH₃)₂OH), 4.72 (se, J = 5.8 Hz, 1 H, CH(CH₃)₂), 6.56 (d, J = 16.2 Hz, 1 H, olefin.), 6.60 (d, J = 3.0 Hz, 1 H, arom.), 6.72 (d, J = 16.2 Hz, 1 H, olefin.), 7.29 (d, J = 3.0 Hz, 1 H, arom.), 7.73 (s, 1 H, arom.), 7.88 (d, J = 8.8 Hz, 1 H, arom.), 8.22 (d, J = 9.0 Hz, 1 H, arom.), 8.53 (s, 1 H, arom.).¹³C-NMR (50 MHz, CD₃OD): δ = 21.8 (2 C), 29.2 (2 C), 42.7, 69.8, 111.9, 112.4, 119.9, 120.4, 125.1, 128.4, 130.6, 134.9, 139.7, 144.4, 144.6, 150.0, 154.0, 159.0. HR ESI-MS: (M + H)⁺calcd. for C₂₀H₂₄N₃O₂: 338.18685, found: 338.18630.

### (E)-tert-Butyl-3-(2-(furan-2-yl)-4-(isopropylamino)quinazolin-6-yl)acrylate (B)

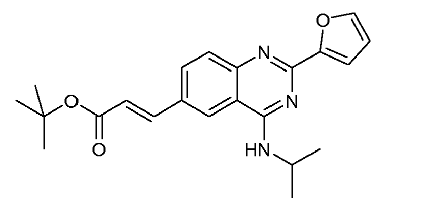

In a Pyrex^{®}-pressure tube a solution of 6-(3-aminophenyl)-2-(furan-2-yl)-N-isopropylquinazolin-4-amine (10) (70 mg, 0.21 mmol), triethylamine (87 µl, 0.62 mmol), tert-butyl acrylate (90.2 µl, 0.62 mmol), tri-o-tolylphosphine (6.3 mg, 21.0 µmol) and palladium acetate (2.3 mg, 10.0 µmol) in acetonitrile (1.0 ml) was heated to 100 °C for 72 h and turned to a dark brown solution. After cooling down to rt the solution was poured in dichloromethane (25 ml). The organic phase was washed with water (15 ml) and brine (15 ml). After drying over Na₂SO₄ the solvent was removed under reduced pressure. The crude product was purified by column chromatography (n-hexane/ethyl acetate 3:1 v/v, R_{f}: 0.21) yielding compound (B) (44 mg, 0.13 mmol, 63%) as a yellow solid, mp.: 127-130 °C. ¹H-NMR (200 MHz, CD₃OD/DMSO-d₆): δ = 2.07 (d, J = 6.6 Hz, 6 H, CH(CH₃)₂), 2.23(s, 9 H, C(CH₃)₃), 5.40 (m, 1 H, CH(CH₃)₂,), 7.29 (d, J = 15.8 Hz, 1 H, olefin.), 7.33 (s, 1 H, arom.), 8.01 (d, J = 3.0 Hz, 1 H, arom.), 8.36 (d, J = 16.2 Hz, 1 H, olefin.), 8.42 (d, J = 8.8 Hz, 1 H, arom.), 8.44, (s, 1 H, arom.), 8.64 (d, J = 7.8 Hz, 1 H, arom.), 9.09 (s, 1 H, arom.). ¹³C-NMR (75 MHz, CD₃OD/DMSO-d₆): δ = 22.5 (2 C), 28.5 (3C), 44.1, 81.7, 113.2, 114.8, 115.3, 121.5, 124.5, 128.4, 132.6, 133.0, 144.0, 146.2, 151.9, 154.2, 155.9, 160.6, 167.7. HR ESI-MS: (M + H)⁺calcd. for C₂₂H₂₆N₃O₃: 380.19742, found: 380.19687.

### 3-(2-(Furan-2-yl)-4-(isoiproipylamino)quinazolin-6-yl)prop-2-yn-l-ol (C)

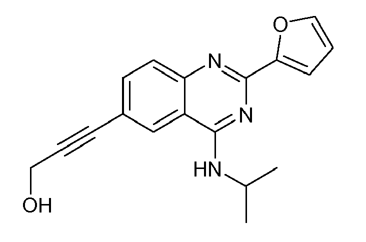

A mixture of DMF (2.0 ml) and triethylamine (0.8 ml, 10.8 mmol) was degassed with ultrasound for 45 min. After addition of 6-(3-aminophenyl)-2-(furan-2-yl)-N-isopropylquinazolin-4-amine (10) (120 mg, 0.32 mmol) and prop-2-yn-1-ol (92 µl, 1.58 mmol) the degassion was continued for 10 min. Afterwards bis(triphenylphosphine) palladium(II)dichloride (6.7 mg, 9.50 µmol), 1,1'-bis(diphenylphosphino)ferrocene (10.5 mg, 19.0 µmol), and copper(I)-iodide (0.6 mg, 3.17 µmol) were added. The catalysts were dried before use. The solution was heated to 50 °C and stirred for 16 h at this temperature (TLC-control). After cooling down to rt the solution was poured in ethyl acetate (10 ml). The organic phase was washed with diluted NH₄Cl (5 ml) solution and brine (5 ml). After drying over Na₂SO₄ the solvent was removed at reduced pressure. The crude product was purified by column chromatography (n-hexane/ethyl acetate 1:2 v/v, R_{f}: 0.35) yielding compound (C) (59 mg, 0.19 mmol, 61 %) as a yellow solid, mp.: 199-200 °C. ¹H-NMR: (300 MHz, CD₃OD): δ = 1.39 (d, J = 6.6 Hz, 6 H, CH(CH₃)₂), 4.70 (se, J = 6.6 Hz, 1 H, CH(CH₃)₂), 4.94 (s, 2 H, CH₂OH), 6.67 (dd, J = 3.6 Hz, J = 3.3 Hz, 1 H, arom.), 7.34 (dd, J = 3.6 Hz, J = 0.9 Hz, 1 H, arom.), 7.76 (m, 3 H, arom.), 8.29 (s, 1 H, arom.). ¹³C-NMR (75 MHz, CD₃OD): δ = 22.3 (2 C), 44.0, 51.2, 84.9, 89.9, 113.1, 114.7, 115.1, 121.2, 127.1, 127.8, 136.4, 146.1, 150.2, 154.2, 155.7, 160.1. HR ESI-MS: (M + H)⁺ calcd. for C₁₈H₁₈N₃O₂: 308.13990, found: 308.13935.

### Example 2. Biological assays

### a) Cell culture and compound treatment (HeLa and RPE cells):

Adherent HeLa cells were routinely passaged and maintained in D-MEM medium supplemented with 10% Fetal Bovine Serum (FBS), 1% Penicillin/Streptomycin and 2 mM L-Glutamine. The cells were cultured at 37 °C; 5% CO₂. hTERT-RPE1 cells were grown in DMEM with 10% fetal bovine serum. HeLa cells constitutively expressing green fluorescent protein-conjugated Histone H2B and mRed fluorescent protein-conjugated α-tubulin were maintained in D-MEM medium supplemented with 10% Fetal Bovine Serum (FBS), 1% Penicillin/Streptomycin, 2 mM L-Glutamine and 0.5 µg/mL Puromycin and cultured at 37 °C; 5% CO2. Compounds (A), (C) and ZM1 were resuspended in DMSO 20 mM stocks and added to the cell culture medium at the final concentrations indicated in the text.

For Western Blot analysis of HH3 phosphorylation levels in mitosis HeLa cells were arrested with 2 mM thymidine for 18 h, released into fresh medium for 6 h, and blocked with 2 µM nocodazole for 16 h. DMSO or the different compounds were added 1 h after thymidine release. Mitotic cells were shaken off and lysed in 50 mM Tris pH 6,8- 2% SDS. Samples were sonicated and boiled. 30 µg protein extract (as determined by Bradford assay, Bio-Rad protein assay) was loaded on SDS-PAGE, transferred to a nitrocellulose membrane and blotted with anti-phospho HH3 (Upstate), anti-Aurora A and anti-tubulin antibodies (Sigma, DM1A).

### b) Treatment of cells and analysis of phenotypes:

For live cell analysis, H2B-GFP/mRFP-αTubulin HeLa cells were treated with DMSO or 2 µM ZM1 or 40 µM (A) or (C). Upon treatment, living cells were added to a humidified chamber at 37 °C with 5% CO₂ attached to a Carl Zeiss microscope (SN:3834000366) equipped with a Hamamatsu EM-CCD digital camera, controlled by AxioVision 4.6 software. Cells were imaged every 3 or 4.5 min (depending on the number of fields filmed) for over 16 h-24 h. Images were taken with the AxioCam MRm 426205 objective and processed using AxioVision software.

For fixed cell analysis, HeLa or H2B-GFP/mRFP-αTubulin HeLa cells were grown on glass coverslips and treated as described above. Cells were fixed in ice cold methanol and mounted in Mowiol. Alternatively, non fluorescent HeLa were treated and subjected to immunofluorescence with anti-tubulin antibodies (Sigma, DM1A) and Hoechst to stain the DNA. Cells were observed on a Leica DM16000B microscope with the HCX PL APO CS 63x 1.4 objective. Images were taken with a Leica DFC 350FX camera, controlled by Leica Application Suit (LAS-AF 6000) software and processed with Adobe Photoshop.

### c) Fluorescence Activated Cell Sorting (FACs):

After treatment, FACS analysis was performed on a BD FACSCanto^{™} Flow Cytometer (Cat.No. 335860 BD Biosciences) using FACSDiva Software. Cells were trypsinized, washed wish PBS and fixed overnight in EtOH (-20°C). After washing with PBS, cells were stained with a propidium iodide solution (PI; 15 µg/mL)/ RNase A (30 µg/mL) for 30 min at 37 °C. RNase was added to avoid signals caused by the binding of PI to RNA.

### d) In vitro Aurora A kinase assay:

The IC₅₀ for the different compounds was determined with Invitrogen Z'-Iyte kinase assay kit Ser/Thr 1 peptide PV3174 following the manufacturer's instructions. In brief, the concentrations of the kinases at the reported *K_{M app}* ATP concentrations (Aurora A: 10 µM ATP, Aurora B: 64 µM ATP) were first optimized using 10-point dose-response curves giving a protein concentration that resulted in approximately 30% phosphorylation. This amount was then used in subsequent assay steps.

Compounds were prepared in 384-well plates (Greiner 384-well PP 781280) at a highest concentration of 400 µM (in 4% DMSO) and then serially diluted in 2-fold steps by hand, keeping the DMSO percentage constant. 1.25 µl of compound solution was subsequently transferred 1:1 to a 384-well assay plate (Greiner 384-well ProxiPlate Plus F 6008260) for screening at a highest concentration in the final assay of 100 µM (in 1% DMSO).

In the first step, 2.5 µl of a solution containing 8 nM Aurora A (batch S74, prepared in-house at EMBL) or 40 nM Aurora B (Invitrogen PV3970) and 4 µM Ser/Thr peptide 1 (Invitrogen PV3174) was added to each well, followed by 1.25 µl of the respective ATP solution (40 µM for Aurora A, 256 µM for Aurora B). The final 5 µl reaction volume consisted of 4 nM Aurora A or 20 nM Aurora B, 2 µM Ser/Thr 1 peptide, 50 mM HEPES pH 7.5, 0.01 % BRIJ-35, 10 mM MgCl₂, and 1 mM EGTA, in addition to 10 µM or 64 µM ATP (for Aurora A and Aurora B, respectively). To simulate 100% inhibition (positive control), buffer was added instead of the ATP solution, while in the 0% inhibition wells compound was replaced by 4% DMSO solution. The plates were then spun down at 1000 rpm for 1 min, followed by incubation at rt for 1 h. In the second step, 2.5 µl of Development solution (Development A reagent diluted 1:2048 in development buffer) was added to each well, followed again by centrifugation at 1000 rpm for 1 min and subsequent incubation at rt for 1 h. In the final step, the reaction in each well was stopped by the addition of 2.5 µl Stop reagent, followed by a brief centrifugation step and immediate readout (PerkinElmer EnVision HTS; ex. 405 nm, coumarin em. 450 nm, fluorescein em. 535 nm).

Percentage phosphorylation was calculated using 0% and 100% phosphorylation controls. The former wells contained 1.25 µl DMSO (4%), 1.25 µl buffer (as above), and 2.5 µl of a 4 µM Ser/Thr 1 peptide solution (without kinase), while in the latter the peptide solution was replaced by a 4 µM solution of a supplied phosphopeptide. The action of the development reagent was assessed using protease controls (standard wells with and without development solution added in step two).

The [coumarin emission/fluorescein emission] ratio was used together with the min. and max. signals at the two wavelengths to yield percentage phosphorylation, from which inhibition was derived (according to manufacturer's instructions). Calculated inhibition values were then fitted to 11-point variable slope sigmoidal dose-response curves using GraphPad Prism software, yielding the compound IC₅₀s.

To determine the IC₅₀ of receptor-tyrosine-kinase an assay based on the ELISA-principle using kinases from Biomol (N-terminally fused to GST), POD-coupled antibody from Calbiochem (PY20) and BM chemiluminescence ELISA substrate from Roche was followed.

In short, a white 96 wells microplate was incubated overnight with 100 µl/well of the substrate solution (poly-Glu-Tyr diluted with PBS to 100 µg/ml) at 4 °C. The solution was removed and the plate was washed twice for 2 min with PBS-T. Afterwards the kinase solution (50 µl/well)( KDR (VEGFR2), 5 ng; Flt4 (VEGFR3), 20 ng; EGFR, 5 ng; IGFR, 15 ng; FGFR, 10 ng and Tie2, 20 ng) and the inhibitor solution (25 µl/well) were added to each well. To perform positive and negative control, 5% aqueous DMSO (25 µl/well) were used instead of inhibitor solution. Addition of ATP solution (25 µl/well) started the reaction, whereas 40 mM MnCl₂ solution (25 µl/well) was added to the negative control instead of ATP. After incubation under mild shaking conditions for 30 min at rt the plate was washed with PBS-T (3 x 2 min) and subsequently incubated with antibody solution (anti-Phosphotyrosine-antibody (POD-coupled) diluted 1:10.000 in PBS-T + 0,2% (m/v) BSA)(100 µl/well) for 1 h again while shaking. In the end the plate was washed with PBS-T (3x5 min) and provided with the chemiluminescence substrate (50 µl/well). After 3 min the occurring luminescence was measured using a luminometer. A positive control was measured without inhibitor to determine the activity of the kinase, a negative control without inhibitor and ATP to abstract the background signal from the results. For determination of IC₅₀ values, the relative luminescence units per second (RLU/s) were blotted against the inhibitor concentrations. In doing so each concentration measurement was done triplicate and at least 5 different inhibitor concentrations were used to determine IC₅₀ value.

### e) Cilia assembly and disassembly assays:

Cilia assembly and disassembly assays were performed as described in Pugacheva et al., Cell 2007, 129, 1351-1363. Briefly, to study cilia assembly hTRET-RPE1 cells were plated at 30% confluence in plates containing glass cover slips, and starved for 48 h (in Opti-MEM) to induce cilia formation in the presence of ZM1, (C) or DMSO (vehicle). For analysis of cilia disassembly, cilia were assembled by 48 h starvation in Opti-MEM, and disassembled by incubation in DMEM-10% serum. Cells were fixed after 2 and 4 h. ZM1, (C) or DMSO (vehicle) were added to hTERT-RPE1 cells 2 h prior to the initiation of cilia disassembly. To visualize the centrosomes and the cilia, fixed cells were immunostained with anti-γ-tubulin (Sigma GTU88) and anti-acetylated-tubulin (Sigma), and DNA was stained with Hoechst.

### f) RTK-Assay (ELISA)

This assay is based on the ELISA-principle using kinases from Biomol (N-terminally fused to GST), POD-coupled antibody from Calbiochem (PY20) and BM chemiluminescence ELISA substrate from Roche.

Each well of a 96 well microplate (white, flat bottom, Greiner, Lumitrac600) was covered with a kinase polypeptide substrate containing glutamate and tyrosine. The substrate was incubated with a kinase, a defined inhibitor concentration and ATP. After kinase inhibitor depending phosphorylation of the substrate a POD-coupled antibody was added that selectively binds to phosphorylated tyrosine. Finally, a chemiluminescence reagent was added and light emission measured using an Orion Microplate Luminometer (Berthold).

The detected counts correlate directly with the amount of phosphorylated tyrosine and indirectly with the activity of the inhibitor.

A positive control was measured without inhibitor to determine the activity of the kinase, a negative control without inhibitor and ATP to abstract the background signal from the results. For determination of IC₅₀ values, the relative luminescence units per second (RLU/s) were blotted against the inhibitor concentrations. In doing so each concentration measurement was done triplicate and at least 5 different inhibitor concentrations were used to determine IC50 value.

The following solutions were prepared for the receptor-tyrosine-kinase assay:
- PBS (10x) 1 g KCl, 1 g KH₂PO₄, 40 g NaCl, 13.2 g Na₂HPO₄, ad 1 l H₂O
- PBST (1x) PBS 10x + 0,5% Tween20 → diluted 1:10
- substrate poly-Glu-Tyr 4:1 (10mg/ml in PBS) → diluted with PBS to 100µg/ml
- kinase buffer 100mM HEPES, 100mM NaCl, 0.1mM Na₃VO₄
- kinase solution kinase buffer, kinase stock solution diluted to reach the following concentration defined in the table

**Table "kinase dilution"**

| kinase | concentration of stock solution | concentration in well |
|---|---|---|
| KDR (VEGFR2) | 76 ng/µl | 5 ng |
| Flt-4 (VEGFR3) | 66 ng/µl | 20 ng |
| EGFR | 108 ng/µl | 5 ng |
| IGFR | 94 ng/µl | 15 ng |
| FGFR | 65 ng/µl | 10 ng |
| Tie2 | 200 ng/µl | 20 ng |

- ATP-solution 100µM ATP in 40mM MnCl₂
40mM MnCl₂ as negative control
- inhibitor solution defined concentrations in 5% aqueous DMSO
5% aqueous DMSO as positive control and negative control
- antibody buffer PBST + 0,2% (m/v) BSA
- antibody solution anti-Phosphotyrosine-antibody (POD-coupled) → dilute 1:10.000 in antibody buffer
- luminescence reagent prepared according to Assay Kit instructions
substrate A : substrate B = 100:1, mixed 15min before use

A white 96 well microplate was incubated overnight with 100µl/well of the substrate solution at 4°C. The solution was removed and the plate was washed twice for 2 minutes with PBST. Afterwards the kinase solution (50µl/well) and the inhibitor solution (25µl/well) were added to each well. To perform positive and negative control, 5% aqueous DMSO (25µl/well) were used instead of inhibitor solution. Addition of ATP solution (25µl/well) started the reaction, whereas 40mM MnCl2 solution (25µl/well) was added to the negative control instead of ATP. After incubation under mild shaking conditions for 30 minutes at room temperature the plate was washed with PBST (3 x 2min) and subsequently incubated with antibody solution (100µl/well) for 1 hour again while shaking. In the end the plate was washed with PBST (3x5min) and provided with the chemiluminescence substrate (50µl/well). After 3 minutes the occurring luminescence was measured using a luminometer.

### Selectivity against other kinases

The derivatives were investigated considering their ability to inhibit different enzymes. For compounds (A) and (C) selective inhibition of Aurora A was shown. In the case of compound (C), Aurora B and different receptor-tyrosine kinases were not inhibited or they were inhibited much weaker than Aurora A.

**Table 1. IC₅₀ values of (A) and (C) against Aurora A, Aurora B and a panel of other selected kinases.**

| | **Aurora A** | **Aurora B** | **EGFR** | **IGFR** | **KDR (VEGFR 2)** | **Flt-4 (VEGFR 3)** | **FGF** | **Tie-2** |
|---|---|---|---|---|---|---|---|---|
| | | | | | | | | |
| **(C)** | 2,75 µM | 3380 µM | x | X | x | x | x | x |
| **(A)** | 1,93 µM | 16,2 µM | x | 41,55 ± 5,85 µM | 8,17 ± 0,87 µM | 4,24 ± 0,77 µM | x | x |

| | | | | | | | | |
|---|---|---|---|---|---|---|---|---|
| x: IC₅₀ > 100 µM | | | | | | | | |

The described compounds were also used for cellular investigations. Their effect to HeLa-cells, a cancer cell line, was investigated. Therefore they were observed by time-lapse video microscopy. Cells transfected with (C) showed an increased amount of 4n DNA. The entrance of the cells to mitosis was investigated. Only 28.8% of the (C)-transfected cells entered mitosis. They were unable to align their chromosomes and died without entering a new interphase. Transfection with (A) lead to an increased cell death rate. The (C)-induced effects indicate Aurora A-inhibition. With an immunofluorescence assay the chromosomes and microtubules were visualized. The (C)-transfected cells showed increased formation of multipolar spindles and other abnormal mitotic structures. These results demonstrate that (C) has strong effects to mitotic progression with phenotypes related to Aurora A-inhibition.

In summary the described derivatives are highly active in the isolated enzyme as well as in cellular systems. Their effects in cellular test systems correlate with inhibition of Aurora A. For the known inhibitors this is not the case, they all show phenotypes of Aurora B-inhibition.

### Compound (C) delays G2/M progression in human tissue culture cells

Previous studies have shown that the treatment of cells with small molecule inhibitors for Aurora kinase resulted in the accumulation of cells with a DNA content of >4n. We first examined by flow cytometry the cell cycle profiles of HeLa cells incubated in medium containing increasing concentrations of any of (A) or (C) for 24 h. As a positive control we used one of the previously characterized Aurora inhibitor ZM447439 (ZM1) at 2 µM.

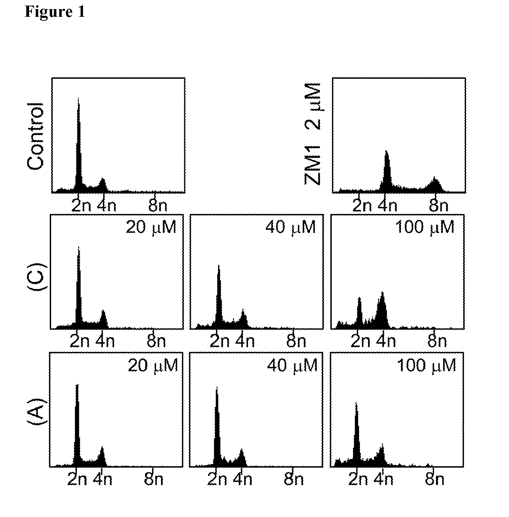

Consistently with published results, treatment with ZM1 increased the proportion of cells with 4n and 8n DNA content (Figure1 and Table 2). HeLa cells incubated with (C) showed a dose dependent increase in the proportion of cells with more than 2n DNA content, although without reaching more than 4n DNA content (Figure1 and Table 2). Compound (A) did not alter the distribution of cells in the different cell cycle stages. Incubation for longer periods of time (48 h) with any of the three compounds resulted in massive cell death.

The high proportion of 8n DNA containing cells observed after ZM1 treatment has been related to Aurora B inhibition, also characterized by a decrease in the levels of histone H3 (HH3) phosphorylation on Ser10. Phosphorylation at this site is frequently used as a marker for Aurora B activity. The lack of 8n cells accumulation following treatment with (A) and (C) suggested that none of them inhibited Aurora B activity in vivo. To confirm this hypothesis we studied the HH3 phosphorylation levels in mitotic cells treated with the different compounds for 20 h.

**Table 2. Percentage of cells in G1, S, G2/M and with more than 4n DNA content calculated from Figure 1.**

| % of cells | | <2n | 2n | 2<n<4 | 4n | >4n |
|---|---|---|---|---|---|---|
| Control | | 6.8 | 64.0 | 15.7 | 13.6 | 0.8 |
| ZM1 2µM | | 3.0 | 1.2 | 2.8 | 51.1 | 43.5 |
| (C) | 20µM | 7.6 | 55.2 | 17.5 | 19.0 | 1.3 |
| | 40µM | 10.5 | 47.2 | 20.0 | 21.2 | 1.8 |
| | 100µM | 10.0 | 21.4 | 25.2 | 41.8 | 1.5 |
| (A) | 20µM | 4.4 | 58.5 | 17.6 | 19.2 | 0.7 |
| | 40µM | 3.8 | 58.5 | 17.7 | 19.9 | 3.8 |
| | 100µM | 13.8 | 48.4 | 15.5 | 20.8 | 2.1 |

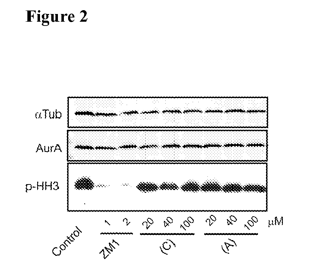

As shown in Figure 2, ZM1 treatment strongly reduced HH3-Ser10 phosphorylation consistently with published data. By contrast, treatment with (A) and (C) had no effect at any of the concentrations tested, supporting the idea that Aurora B activity is unaffected in vivo.

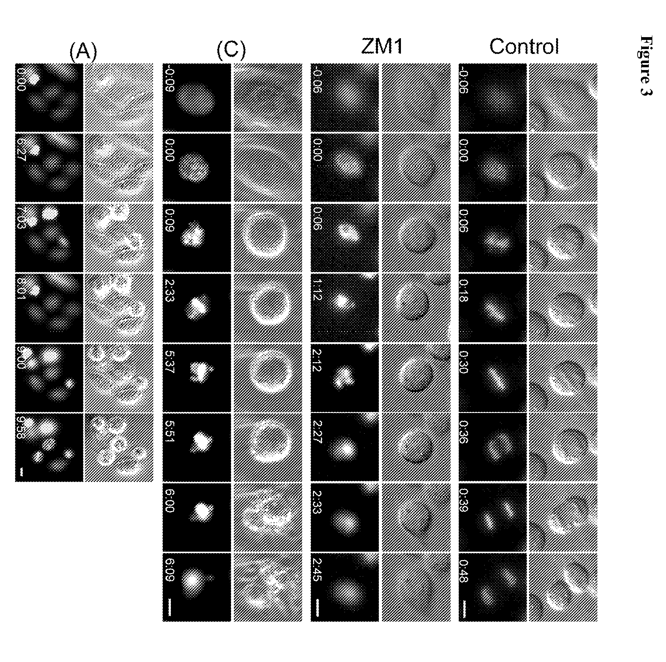

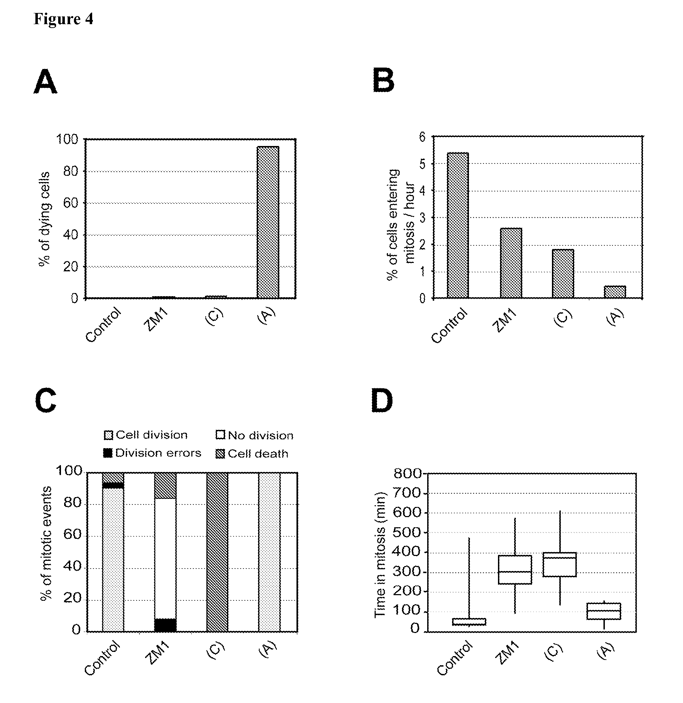

The increase in the proportion of cells with more than 2n DNA content could be due to several causes: several cycles of DNA replication or cytokinesis errors as shown for cells treated with other Aurora inhibitors. To determine the primary cause for the phenotype produced by (C) and examine whether (A) may interfere with mitosis, we examined live cells by time-lapse video microscopy. To visualize DNA and microtubules we used a HeLa stable cell line expressing H2B-GFP and mRFP-αTubulin. Cells were placed in medium containing 2 µM ZM1 or 40 µM of any of our three compounds and imaged every 3 or 4.5 min for 16 h-24 h (Figure 3). The resulting time-lapse images were used to determine the following parameters: (1) the percentage of cell death among non dividing cells (Figure 4A), (2) the percentage of cells entering mitosis (Figure 4B), (3) the outcome of mitosis: normal division (cell division), return to interphase without chromatin segregation and without dividing (no division), cytokinesis failure (division errors), cell death without resolving mitosis (cell death) (Figure 4C), and (4) the length of time spent in mitosis (TIM), defined as the interval between DNA condensation and anaphase resolution (Figure 4D).

Control cells, plated in medium containing DMSO, were healthy throughout the observation time. An average of 5.4 % of the cells entered mitosis per hour (Figure 4B) and almost all of them (90.3%) were able to divide successfully with an average TIM of 39 min (median) (Figure 4D). Treatment with ZM1 decreased by half the number of cells entering mitosis. In agreement with published data, it also increased the TIM to 306 min. During their time in mitosis, cells had problems in reaching metaphase, and 76% of them de-condensed their DNA and went back to interphase without chromosome segregation. Incubation of cells with (C) also reduced the number of cells entering mitosis and increased the TIM (median 375 min), but unlike cells treated with ZM1, all the (C) treated cells died without returning to interphase or right after an asymmetric segregation.

In addition to these mitotic phenotypes, we observed that (A) induced a massive cell death (Figure 4A), so by 11 h of incubation almost no cells remained in the dish. During this period of observation the few cells that entered mitosis completed it successfully.

It has been reported that inhibition of Aurora B kinase activity by over expressing a dead kinase mutant produces an increase in the proportion of cells with 4n and 8n content, due to a failure in cell division. By contrast, over expression of a kinase dead Aurora A does not affect the DNA content profile. The fact that ZM1 mimics Aurora B phenotype supports the in vivo Aurora B inhibition by this compound. Our data suggest that (C) does not inhibit Aurora B in vivo and show that it induces defects compatible with Aurora A inhibition.

Compound (A) had a strong effect on cell viability independent of mitosis, without showing other defects that could suggest an inhibition of Aurora kinases.

### Compound (C) interferes with bipolar spindle formation

To get a better idea about the defects causing cell division failure in cells treated with (C), we examined fixed cells to better visualize the chromosomes and the microtubules. The stable HeLa cells expressing GFP-Histone and mRed-tubulin were incubated with 40 µM (C), 2 µM ZM1 or DMSO (Control) for 24 h, fixed in cold methanol and mounted. In parallel, standard HeLa cells were treated in the same way. The cells were fixed and processed for immunofluorescence with anti-tubulin antibodies to visualize the microtubules and Hoechst to stain the DNA. Mitotic cells were examined and classified into the different mitotic phases and other abnormal figures as shown in Figure 5A.

Cells incubated with ZM1 showed a strong increase of the number of mitotic cells showing chromosome alignment defects in metaphase: although most chromosomes aligned on the metaphase plate, several were in the proximity of the spindle poles (Figure 5). A high percentage of cells were multinucleated. These data are consistent with the reported phenotypes for Aurora B inhibition or silencing.

By contrast, cells treated with (C) showed a large proportion of abnormal mitotic spindles (defined as those that did not display canonical bipolar spindle formation: monopolar, multipolar and other aberrant structures).

We conclude that (C) has a strong effect on mitotic progression and induces phenotypes that are compatible with Aurora A inhibition.

### Compound (C) impairs cilia disassembly

hTERT-RPE1 cells were plated at 50% confluence in medium without serum. After 48 h more than 80% of the cells had a clearly visible cilia as detected by immunofluorescence with an anti acetylated α-tubulin antibody. As previously described changing the culture medium to one containing 10% fetal bovine serum caused cilia disassembly in the first two hours after serum stimulation (Figure 6). Pretreatment of cells with ZM1 1-2 µM did not have any effect on cilia disassembly. By contrast it was strongly inhibited in cells pretreated with 40 µM (C) (Figure 6).

These results show that compound (C) promoted a mitotic arrest compatible with Aurora A inhibition in vivo. To determine more accurately whether this compound inhibits specifically Aurora A we have examined whether it inhibited the disassembly of the primary cilia, a process that has been shown to be specifically regulated by Aurora A. Our results show that compound (C) indeed inhibits this process indicating that the compound inhibits Aurora A in vivo.

## Claims

1. A compound of formula (I), a stereoisomer thereof, a pharmaceutically acceptable salt thereof or a pharmaceutically acceptable solvate thereof: 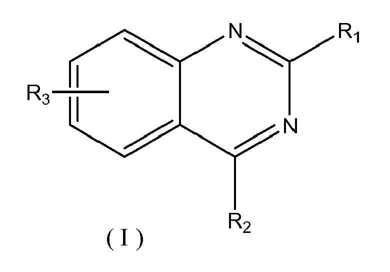 wherein:
R₁ is a radical of one of the known ring systems with 1-2 rings, wherein each one of the rings forming said ring system
has 3-7 members, each member independently selected from C, N, O, S, CH, CH₂, NH,
is saturated, partially unsaturated or aromatic, and
is partially or totally fused;
being each ring forming part of the ring system optionally substituted by at least one radical selected from the group consisting of: halogen, nitro, cyano, linear or branched (C₁-C₆)alkyl, halo-(C₁-C₆)alkyl, linear or branched (C₂-C₆)alkenyl, -OR₆, -COR₆, -COOR₆, -OC(O)R₆, -C(O)NR₄R₅, -NR₄R₅, -R₇NHR₈, -SR₆, -SO-R₆, and -SO₂-R₆;
R₄, R₅, R₆, and R₈ are radicals, same or different, independently selected from the group consisting of hydrogen, and linear or branched (C₁-C₆)alkyl;
R₇ is a biradical selected from the group consisting of: linear or branched (C₁-C₆)alkyl; linear or branched (C₂-C₆)alkenyl; and linear or branched (C₂-C₆)alkynyl;
R₂ is a radical selected from the group consisting of -NHR₉, -C≡C-CR₁₀R₁₁R₁₂, (*E*)-CH=CH-CR₁₀R₁₁R₁₂, and (Z)-CH=CH-CR₁₀R₁₁R₁₂;
R₉ is a radical selected from the group consisting of:
linear or branched (C₁-C₆)alkyl optionally substituted by at least one radical selected from the group consisting of halogen, -OH, -SH, nitro, cyano, -NH₂, (C₁-C₄)alkyl, halo-(C₁-C₆)alkyl, -O-(C₁-C₆)alkyl, -S-(C₁-C₆)alkyl, -COR₁₃, -COOR₁₃, -OC(O)R₁₃, -SOR₁₃, -SOOR₁₃, -OS(O)R₁₃; and
a known ring system having 3-6 members, each member independently selected from C, N, O, S, CH, CH₂, and NH, wherein the ring is saturated, partially unsaturated or aromatic, optionally substituted by at least one radical selected from the group consisting of halogen, -OH, -SH, nitro, cyano, amino, linear or branched (C₁-C₄)alkyl, halo-(C₁-C₆)alkyl, -O-(C₁-C₆)alkyl,-S-(C₁-C₆)alkyl, -COR₁₄, -COOR₁₄, -OC(O)R₁₄, -SOR₁₄, -SOOR₁₄, -OS(O)R₁₄;
R₁₀, R₁₁, and R₁₂ are radicals, same or different, independently selected from the group consisting of hydrogen, halogen, linear or branched (C₁-C₆)alkyl, -NH₂, -OH, -SH, -NR₁₅R₁₆, halO-(C₁-C₆)alkyl, -OR₁₇, -SR₁₇, -COR₁₇, -COOR₁₇, -OC(O)R₁₇, -SOR₁₇, -SOOR₁₇, -OS(O)R₁₇, and -C(O)NR₁₅R₁₆;
R₁₃, R₁₄, R₁₅, R₁₆ and R₁₇ are radicals, same or different, independently selected from the group consisting of hydrogen, and linear or branched (C₁-C₄)alkyl;
R₃ is a radical selected from the group consisting of -C≡C-CR₁₈R₁₉R₂₀, (*E*)-CH=CH-CR₁₈R₁₉R₂₀, and (*Z*)-CH=CH-CR₁₈R₁₉R₂₀;
R₁₈, R₁₉, and R₂₀ are radicals, same or different, independently selected from the group consisting of hydrogen, halogen, linear or branched (C₁-C₆)alkyl, -NH₂, -OH, -SH, -NR₂₁R₂₂, halo-(C₁-C₆)alkyl, -OR₂₃, -SR₂₃, -COR₂₃, -COOR₂₃, -OC(O)R₂₃, -SOR₂₃, -SOOR₂₃, -OS(O)R₂₃, and -C(O)NR₂₁R₂₂;
R₂₁, R₂₂, and R₂₃ are radicals, same or different, independently selected from the group consisting of hydrogen, and linear or branched (C₁-C₄)alkyl;
with the proviso that compound (I) is not
6-ethynyl-2-(1H-imidazol-1-yl)-N-(2-methoxyethyl)quinazolin-4-amine or 2-(2-(2-(1H-imidazol-1-yl)-6-(prop-1-ynyl)quinazolin-4-ylamino)ethoxy)ethanol

2. The compound according to claim 1, wherein R₁ is a radical of a known ring system consisting of one ring which is saturated, partially unsaturated or aromatic, has from 5-6 members, each member is independently selected from C, N, O, S, CH, CH₂, NH;
being the radical optionally substituted by at least one radical selected from the group consisting of: halogen, nitro, cyano, linear or branched (C₁-C₆)alkyl, halo-(C₁-C₆)alkyl, linear or branched (C₂-C₆)alkenyl, -OR₆, -COR₆, -COOR₆, -OC(O)R₆, -C(O)NR₄R₅, -NR₄R₅, -R₇NHR₈, -SR₆, -SO-R₆, and -SO₂-R₆;
R₄, R₅, R₆, and R₈ are radicals, same or different, independently selected from the group consisting of hydrogen, and linear or branched (C₁-C₆)alkyl;
R₇ is a biradical selected from the group consisting of: linear or branched (C₁-C₆)alkyl; linear or branched (C₂-C₆)alkenyl; and linear or branched (C₂-C₆)alkynyl.

3. The compound according to any of claims 1-2, wherein R₁ is a radical selected from 2-furyl, 3-furyl, 2-thiophenyl, 3-thiophenyl, 2-thiazolyl, 4-thiazolyl, 5-thiazolyl, 2-oxazolyl, 4-oxazolyl, 5-oxazolyl, 3-isothiazolyl, 4-isothiazolyl, 5-isothiazolyl, 2-pyrrolyl, 3-pyrrolyl, 2-imidazolyl, 4-imidazolyl, 5-imidazolyl, 1-pyrazolyl, 3-isoxazolyl, 4-isoxazolyl, 2-pyridinyl, 3-pyridinyl, 4-pyridinyl, pyrazinyl, and phenyl; being the radical optionally substituted by at least one radical selected from the group consisting of: halogen, nitro, cyano, linear or branched (C₁-C₆)alkyl, halo-(C₁-C₆)alkyl, linear or branched (C₂-C₆)alkenyl, -OR₆, -COR₆, -COOR₆, -OC(O)R₆, -C(O)NR₄R₅, -NR₄R₅, -R₇NHR₈, -SR₆, -SO-R₆, and -SO₂-R₆;
R₄, R₅, R₆, and R₈ are radicals, same or different, independently selected from the group consisting of hydrogen, and linear or branched (C₁-C₆)alkyl;
R₇ is a biradical selected from the group consisting of: linear or branched (C₁-C₆)alkyl; linear or branched (C₂-C₆)alkenyl; and linear or branched (C₂-C₆)alkynyl.

4. The compound according to any of claims 1-3, wherein R₂ is a -NHR₉; being R₉ a radical selected from the group consisting of:
linear or branched (C₁-C₆)alkyl optionally substituted by at least one radical selected from the group consisting of halogen, -OH, -SH, nitro, cyano, -NH₂, (C₁-C₄)alkyl, halo-(C₁-C₆)alkyl, -O-(C₁-C₆)alkyl, and -S-(C₁-C₆)alkyl, -COR₁₃, -COOR₁₃, -OC(O)R₁₃, -SOR₁₃, -SOOR₁₃, -OS(O)R₁₃; and
a known ring system having 3-6 members, each member independently selected from C, N, O, S, CH, CH₂, and NH, wherein the ring is saturated, partially unsaturated or aromatic, optionally substituted by at least one radical selected from the group consisting of halogen, -OH, -SH, nitro, cyano, amino, linear or branched (C₁-C₄)alkyl, halo-(C₁-C₆)alkyl, -O-(C₁-C₆)alkyl, -S-(C₁-C₆)alkyl, -COR₁₄, -COOR₁₄, -OC(O)R₁₄, -SOR₁₄, -SOOR₁₄, -OS(O)R₁₄;
R₁₃, and R₁₄ are radicals, same or different, independently selected from the group consisting of hydrogen, and linear or branched (C₁-C₄)alkyl.

5. The compound according to any of claims 1-4, wherein R₂ is a -NHR₉; being R₉ a radical selected from the group consisting of:
methyl, ethyl, propyl, isopropyl, butyl, isobutyl, s-butyl, t-butyl, cyclopropyl, cyclobutyl, cyclopentyl, cyclohexyl, 2H-pyran-3-yl, 2-thienyl, 3-thienyl, 2-furyl, 3-furyl, 2-thiophenyl, 3-thiophenyl, 2-thiazole, 4-thiazole, 5-thiazole, 2-oxazole, 4-oxazole, 5-oxazole, 3-isothiazole, 4-isothiazole, 5-isothiazole, 2-pyrrole, 3-pyrrole, 2-imidazole, 4-imidazole, 5-imidazole, 1-pyrazolyl, 3-pyrazolyl, 2-pyridyl, 3-pyridyl, 4-pyridyl, pyrazinyl, 2-pyrimidinyl, and phenyl; being the radical optionally substituted by at least one radical selected from the group consisting of halogen, -OH, -SH, nitro, cyano, amino, linear or branched (C₁-C₄)alkyl, halo-(C₁-C₆)alkyl, -O-(C₁-C₆)alkyl,-S-(C₁-C₆)alkyl, -COH, -COOH, -CO(C₁-C₄)alkyl, -COO(C₁-C₄)alkyl, -OC(O)(C₁-C₄)alkyl, -SOH, -SO₂H, -SO(C₁-C₄)alkyl, -SO₂(C₁-C₄)alkyl, -OS(O)(C₁-C₄)alkyl.

6. The compound according to any of claims 1-3, wherein R₂ is a radical selected from the group consisting -C≡C-CR₁₀R₁₁R₁₂, (*E*)-CH=CH-CR₁₀R₁₁R₁₂, and (*Z*)-CH=CH-CR₁₀R₁₁R₁₂; wherein:
R₁₀, R₁₁, and R₁₂ are radicals, same or different, independently selected from the group consisting of hydrogen, halogen, linear or branched (C₁-C₆)alkyl, -NH₂, -OH, -SH, -NR₁₅R₁₆, halo-(C₁-C₆)alkyl, -OR₁₇, -SR₁₇, -COR₁₇, -COOR₁₇, -OC(O)R₁₇, -SOR₁₇, -SOOR₁₇, -OS(O)R₁₇, and -C(O)NR₁₅R₁₆;
R₁₅, P₁₆ and R₁₇ are radicals, same or different, independently selected from the group consisting of hydrogen, and linear or branched (C₁-C₄)alkyl.

7. The compound according to claim 6, wherein R₂ is a radical selected from the group consisting -C≡C-CR₁₀R₁₁R₁₂, (*E*)-CH=CH-CR₁₀R₁₁R₁₂, and (*Z*)-CH=CH-CR₁₀R₁₁R₁₂; wherein:
at least one of R₁₀, R₁₁ and R₁₂ is hydrogen; and the other R₁₀, R₁₁ or R₁₂ are radicals, same or different, independently selected from the group consisting of hydrogen, halogen, methyl, ethyl, propyl, isopropyl, butyl, isobutyl, s-butyl, t-butyl, -OH, -SH, -NR₁₃R₁₄, trifluoromethyl, trifluoroethyl, methoxyl, ethoxyl, thiomethyl, thioethyl, -COH, -COOH, -COCH₃, -COOCH₃, -OC(O)CH₃, -SOH, -SO₂H, -SOCH₃, -SO₂CH₃, -OS(O)CH₃, and -C(O)NR₁₃R₁₄;
being R₁₃ and R₁₄, same or different, independently selected from hydrogen, methyl, ethyl, propyl, and isopropyl.

8. The compound according to any of claims 1-7, wherein R₃ is a radical selected from the group consisting of -C≡C-CR₁₈R₁₉R₂₀, (*E*)-CH=CH-CR₁₈R₁₉R₂₀, and (*Z*)-CH=CH-CR₁₈R₁₉R₂₀, wherein:
at least one of R₁₈, R₁₉ and R₂₀ is hydrogen; and the other R₁₈, R₁₉ or R₂₀ are radicals, same or different, independently selected from the group consisting of hydrogen, halogen, methyl, ethyl, propyl, isopropyl, butyl, isobutyl, s-butyl, t-butyl, -OH, -SH, -NR₂₁R₂₂, trifluoromethyl, trifluoroethyl, methoxyl, ethoxyl, thiomethyl, thioethyl, -COH, -COOH, -COCH₃, -COOCH₃, -OC(O)CH₃, -SOH, -SO₂H, -SOCH₃, -SO₂CH₃, -OS(O)CH₃, and -C(O)NR₂₁R₂₂;
R₂₁ and R₂₂ are radicals, same or different, independently selected from the group consisting of hydrogen, and linear or branched (C₁-C₄)alkyl.

9. The compound according to any of claims 1-8, wherein:
R₁ is a radical selected from 2-furyl, 3-furyl, 2-thiophenyl, 3-thiophenyl, 2-thiazolyl, 4-thiazolyl, 5-thiazolyl, 2-oxazolyl, 4-oxazolyl, 5-oxazolyl, 3-isothiazolyl, 4-isothiazolyl, 5-isothiazolyl, 2-pyrrolyl, 3-pyrrolyl, 3-isoxazolyl, 4-isoxazolyl and phenyl; being the radical optionally substituted by at least one radical selected from the group consisting of: halogen, nitro, cyano, linear or branched (C₁-C₆)alkyl, halo-(Cₗ₁-C₆)alkyl, linear or branched (C₂-C₆)alkenyl, -OR₆, -COR₆, -COOR₆, -OC(O)R₆, -C(O)NR₄R₅, -NR₄R₅, -R₇NHR₈, -SR₆, -SO-R₆, and -SO₂-R₆;
R₄, R₅, R₆, and R₈ are radicals, same or different, independently selected from the group consisting of hydrogen, and linear or branched (C₁-C₆)alkyl;
R₇ is a biradical selected from the group consisting of: linear or branched (C₁-C₆)alkyl; linear or branched (C₂-C₆)alkenyl; and linear or branched (C₂-C₆)alkynyl;
R₂ is a -NHR₉; being R₉ a radical selected from the group consisting of:
methyl, ethyl, propyl, isopropyl, butyl, isobutyl, s-butyl, t-butyl, cyclopropyl, cyclobutyl, cyclopentyl, cyclohexyl, 2H-pyran-3-yl, 2-thienyl, 3-thienyl, 2-furyl, 3-furyl, 2-thiophenyl, 3-thiophenyl, 2-thiazole, 4-thiazole, 5-thiazole, 2-oxazole, 4-oxazole, 5-oxazole, 3-isothiazole, 4-isothiazole, 5-isothiazole, 2-pyrrole, 3-pyrrole, 2-imidazole, 4-imidazole, 5-imidazole, 1-pyrazolyl, 3-pyrazolyl, 2-pyridyl, 3-pyridyl, 4-pyridyl, pyrazinyl, 2-pyrimidinyl, and phenyl; being the radical optionally substituted by at least one radical selected from the group consisting of halogen, -OH, -SH, nitro, cyano, amino, linear or branched (C₁-C₄)alkyl, halo-(C₁-C₆)alkyl, -O-(C₁-C₆)alkyl,-S-(C₁-C₆)alkyl, -COH, -COOH, -CO(C₁-C₄)alkyl, -COO(C₁-C₄)alkyl, -OC(O)(C₁-C₄)alkyl, -SOH, -SO₂H, -SO(C₁-C₄)alkyl, -SO₂(C₁-C₄)alkyl, -OS(O)(C₁-C₄)alkyl;
R₃ is a radical selected from the group consisting of -C≡C-CR₁₈R₁₉R₂₀, (*E*)-CH=CH-CR₁₈R₁₉R₂₀, and (*Z*)-CH=CH-CR₁₈R₁₉R₂₀, wherein:
at least one of R₁₈, R₁₉ and R₂₀ is hydrogen; and the other R₁₈, R₁₉ or R₂₀ are radicals, same or different, independently selected from the group consisting of hydrogen, halogen, methyl, ethyl, propyl, isopropyl, butyl, isobutyl, s-butyl, t-butyl, -OH, -SH, -NR₂₁R₂₂, trifluoromethyl, trifluoroethyl, methoxyl, ethoxyl, thiomethyl, thioethyl, -COH, -COOH, -COCH₃, -COOCH₃, -OC(O)CH₃, -SOH, -SO₂H, -SOCH₃, -SO₂CH₃, -OS(O)CH₃, and -C(O)NR₂₁R₂₂;
R₂₁ and R₂₂ are radicals, same or different, independently selected from the group consisting of hydrogen, and linear or branched (C₁-C₄)alkyl.

10. The compound according to any of claims 1-9, wherein:
R₁ is a radical selected from 2-furyl, 3-furyl, 2-thiophenyl, and 3-thiophenyl; being the radical optionally substituted by at least one radical selected from the group consisting of: halogen, nitro, cyano, linear or branched (C₁-C₆)alkyl, halo-(C₁-C₆)alkyl, linear or branched (C₂-C₆)alkenyl, -OR₆, -COR₆, -COOR₆, -OC(O)R₆, -C(O)NR₄R₅, -NR₄R₅, -R₇NHR₈, -SR₆, -SO-R₆, and -SO₂-R₆;
R₄, R₅, R₆, and R₈ are radicals, same or different, independently selected from the group consisting of hydrogen, and linear or branched (C₁-C₆)alkyl;
R₇ is a biradical selected from the group consisting of: linear or branched (C₁-C₆)alkyl; linear or branched (C₂-C₆)alkenyl; and linear or branched (C₂-C₆)alkynyl;
R₂ is a -NHR₉; being R₉ a radical selected from the group consisting of:
methyl, ethyl, propyl, isopropyl, butyl, isobutyl, s-butyl, t-butyl, cyclopropyl, cyclobutyl, cyclopentyl, cyclohexyl, and phenyl; being the radical optionally substituted by at least one radical selected from the group consisting of halogen, -OH, -SH, nitro, cyano, amino, linear or branched (C₁-C₄)alkyl, halo-(C₁-C₆)alkyl, -O-(C₁-C₆)alkyl,-S-(C₁-C₆)alkyl, -COH, -COOH, -CO(C₁-C₄)alkyl, -COO(C₁-C₄)alkyl, -OC(O)(C₁-C₄)alkyl, -SOH, -SO₂H, -SO(C₁-C₄)alkyl, -SO₂(C₁-C₄)alkyl, -OS(O)(C₁-C₄)alkyl;
R₃ is a radical selected from the group consisting of -C≡C-CR₁₈R₁₉R₂₀, (*E*)-CH=CH-CR₁₈R₁₉R₂₀, and (*Z*)-CH=CH-CR₁₈R₁₉R₂₀, wherein:
at least one of R₁₈, R₁₉ and R₂₀ is hydrogen; and the other R₁₈, R₁₉ or R₂₀ are radicals, same or different, independently selected from the group consisting of hydrogen, halogen, methyl, ethyl, propyl, isopropyl, butyl, isobutyl, s-butyl, t-butyl, -OH, -SH, -NR₂₁R₂₂, trifluoromethyl, trifluoroethyl, methoxyl, ethoxyl, thiomethyl, thioethyl, -COH, -COOH, -COCH₃, -COOCH₃, -OC(O)CH₃, -SOH, -SO₂H, -SOCH₃, -SO₂CH₃, -OS(O)CH₃, and -C(O)NR₂₁R₂₂;
R₂₁ and R₂₂ are radicals, same or different, independently selected from the group consisting of hydrogen, and linear or branched (C₁-C₄)alkyl.

11. The compound according to any of claims 1-10, which is selected from:
(*E*)-4-(2-(Furan-2-yl)-4-(isopropylamino)quinazolin-6-yl)-2-methylbut-3-en-2-ol ; and
3-(2-(Furan-2-yl)-4-(isopropylamino)quinazolin-6-yl)prop-2-yn-1-ol.

12. A pharmaceutical composition comprising a therapeutically effective amount of a compound of formula (I) as defined in any of claims 1-11 or a pharmaceutically acceptable salt thereof together with the appropriate amounts of pharmaceutically acceptable excipients, carriers, or mixtures thereof.

13. A compound according to any of claims 1-11, or a pharmaceutically acceptable salt thereof, for use as a medicament.

14. A compound according to any of claims 1-11, or a pharmaceutically acceptable salt thereof, for use in the treatment of a disease mediated by Aurora A kinase, including
6-ethynyl-2-(1H-imidazol-1-yl)-N-(2-methoxyethyl)quinazolin-4-amine or 2-(2-(2-(1H-imidazol-1-yl)-6-(prop-1-ynyl)quinazolin-4-ylamino)ethoxy)ethanol

15. The compound according to claim 14, wherein the disease mediated by Aurora A kinase is cancer.

16. The compound according to claim 15, wherein the cancer is selected from the group consisting of colorectal, breast, lung, prostate, pancreatic, bladder, and renal cancer or leukemias or lymphomas.
